# EUROPEAN PATENT SPECIFICATION

(11) **EP 2 176 400 B1**
(45) Date of publication and mention of the grant of the patent: **14.10.2015**
(21) Application number: 08763702.1
(22) Date of filing: 10.07.2008
(51) Int. Cl.: A61K 35/12, C12N 5/0775

(54) **ENCAPSULATED MESENCHYMAL STEM CELLS AND USES THEREOF**
VERKAPSELTE MESENCHYMALE STAMMZELLEN UND VERWENDUNGEN DAVON
CELLULES SOUCHES MÉSENCHYMATEUSES ENCAPSULÉES ET LEURS PROCÉDÉS D'UTILISATION

(30) Priority: 11.07.2007 US 929747 P
(43) Date of publication of application: 21.04.2010
(73) Proprietor: Technion Research & Development Foundation Ltd., 32000 Haifa (IL)
(72) Inventor: MACHLUF, Marcelle, 34608 Haifa (IL); GOREN, Amit, 36740 Nesher (IL)
(74) Representative: Dennemeyer & Associates S.A.
(86) International application number: PCT/IL2008/000962
(87) International publication number: WO 2009/007979

(56) References cited:
- EP-A- 1 854 455
- WO-A-2007/136760
- WO-A-2008/078157
- US-A1- 2007 116 680
- WEBER M ET AL: "Formation of cartilage matrix proteins by BMP-transfected murine mesenchymal stem cells encapsulated in a novel class of alginates" BIOMATERIALS, ELSEVIER SCIENCE PUBLISHERS BV., BARKING, GB, vol. 23, no. 9, 1 May 2002 (2002-05-01), pages 2003-2013, XP004345246 ISSN: 0142-9612 cited in the application
- JOKI T ET AL: "Continuous release of endostatin from microencapsulated engineered cells for tumor therapy" NATURE BIOTECHNOLOGY, NATURE PUBLISHING GROUP, NEW YORK, NY, US, vol. 19, no. 1, 1 January 2001 (2001-01-01), pages 35-39, XP002244221 ISSN: 1087-0156
- ABBAH ET AL: "In vitro evaluation of alginate encapsulated adipose-tissue stromal cells for use as injectable bone graft substitute" BIOCHEMICAL AND BIOPHYSICAL RESEARCH COMMUNICATIONS, ACADEMIC PRESS INC. ORLANDO, FL, US, vol. 347, no. 1, 18 August 2006 (2006-08-18), pages 185-191, XP005534439 ISSN: 0006-291X
- CHAN ET AL: "Self-assembled collagen-human mesenchymal stem cell microspheres for regenerative medicine" BIOMATERIALS, ELSEVIER SCIENCE PUBLISHERS BV., BARKING, GB, vol. 28, no. 31, 18 August 2007 (2007-08-18), pages 4652-4666, XP022207006 ISSN: 0142-9612
- DING ET AL: "Biologic effect and immunoisolating behavior of BMP-2 gene-transfected bone marrow-derived mesenchymal stem cells in APA microcapsules" BIOCHEMICAL AND BIOPHYSICAL RESEARCH COMMUNICATIONS, ACADEMIC PRESS INC. ORLANDO, FL, US, vol. 362, no. 4, 15 September 2007 (2007-09-15), pages 923-927, XP022249531 ISSN: 0006-291X
- Julia F. Markusen ET AL: "Behavior of Adult Human Mesenchymal Stem Cells Entrapped in Alginate-GRGDY Beads", Tissue Engineering, vol. 12, no. 4, 1 April 2006 (2006-04-01), pages 821-830, XP55000136, ISSN: 1076-3279, DOI: 10.1089/ten.2006.12.821
- ABBAH S A ET AL: "In vitro evaluation of alginate encapsulated adipose-tissue stromal cells for use as injectable bone graft substitute", BIOCHEMICAL AND BIOPHYSICAL RESEARCH COMMUNICATIONS, ACADEMIC PRESS INC. ORLANDO, FL, US, vol. 347, no. 1, 18 August 2006 (2006-08-18), pages 185-191, XP024925485, ISSN: 0006-291X, DOI: DOI:10.1016/J.BBRC.2006.06.072 [retrieved on 2006-08-18]
- Amanda W. Lund ET AL: "Osteogenic differentiation of mesenchymal stem cells in defined protein beads", Journal of Biomedical Materials Research Part B: Applied Biomaterials, vol. 87B, no. 1, 1 October 2008 (2008-10-01), pages 213-221, XP055192356, ISSN: 1552-4973, DOI: 10.1002/jbm.b.31098

## Description

The present invention relates to a method of producing encapsulated primary mesenchymal stem cells according to claim 1, a composition of matter according to claim 2, a method of ex-vivo proliferating and/or differentiating mesenchymal stem cells according to claim 3, a pharmaceutical composition according to claim 5.

Stem cells have excellent potential for being the ultimate source of transplantable cells for many different tissues. These cells seem to have an unlimited capacity to propagate in culture conditions, while remaining undifferentiated. Furthermore, upon differentiation, these cells can transform into various cell types. Stem cells have also been tested successfully for genetic manipulation and gene targeting, and could be used for cell-based therapy.

To date, there is insufficient information regarding the relationship between stem cells and the immune system. It is also unclear whether this relationship is directed by cell-to-cell interactions or by environmental factors shaping stem cell phenotype and functions. It is known that embryonic stem cells (ESCs) and adult mesenchymal stem cells (MSCs) have limited immunogenicity and the latter have been shown to have immunoregulatory influence on the immune system. Both ESCs and MSCs exhibit low levels of major histocompatibility complex (MHC)-I molecules, and do not exhibit MHC-II and the co-stimulatory molecules such as CD80, CD86 and CD40. It has also been shown that ESCs and MSCs produce a variety of growth factors, cytokines, chemokines and proteases that can influence the immunoregulation and immunogenicity of these cells.

The entrapment of viable cells within semi-permeable membranes (cell microencapsulation) is one of the most important approaches for cell-based therapy and the continuous delivery of drugs and proteins. The cell immobilization devices are implanted near the target organ so that the entrapped cells produce the desired therapeutic effect while the polymer membrane isolates them from the physiological environment. The permeable polymer membrane enables the entrance of small molecules, such as nutrients and oxygen which are essential for the growth and viability of the entrapped cells, while preventing the escape of the cells and the entrance of molecules larger than a specific critical size, such as antibodies and other components of the immune system. Cell microencapsulation has several advantages over other sustained release systems: ease of production, a larger surface area to volume ratio, continuous delivery of the therapeutic factor as long as the cells are alive, synthesis of the therapeutic factors (mainly proteins) is performed *in situ*, and it overcomes problems associated with protein stability and capsule capacity limitations.

The cell source for transplantation is a very important factor when considering graft rejection. Autologous cell sources are extremely limited and difficult to expand *ex vivo*. Alternative sources such as allograft or xenograft cells cannot be implanted without the use of immunosuppressant. Immunosuppression is associated with deleterious side effects, such as increased susceptibility to viral, fungal, and bacterial infections, and increased risk for the development of malignancies. Immunoprotection by encapsulation can theoretically enable transplantation of allogenic and xenogenic cells in the absence of immunosuppresion. Nevertheless, most of the encapsulated cells which originate from allogenic or xenogenic sources (especially islet cells) eventually cause an immune rejection by the host. These immune rejections are mainly due to shed antigens originating from the encapsulated cells. Shed antigens occur for several reasons, including death of encapsulated cells and chemotaxis. Local attraction of macrophages and natural killer (NK) cells by shedding is more apparent for xenografts than for allografts. Xenogenic antigen release attracts and activates macrophages, NK cells and dendritic cells, which in turn release cytokines and oxygen radicals. Pro-inflammatory mediators such as interleukin 1 (IL-1) and tumor necrosis factor (TNF) induce the production of chemoattractant factors called chemokines, which attract more macrophages to the implanted area.

Dean SK et al., (Transplantation. 2006, 82: 1175-84) showed that encapsulated ESCs can differentiate towards all three cell lineages when transplanted *in vivo.*

Perrot P, et al. [Ann Plast Surg. 2007 Aug; 59(2):201-6] showed that implantation of encapsulated MSC in alginate beads resulted in a mineralization process characterized by lamellar mature bone with osteocytes after 10 weeks.

Abbah SA et al., (J. Mater. Sci. Med. 2008, 19:2113-2119) describe the encapsulation of bone marrow stromal cells in alginate microcapsules.

Additional background art includes Weber M, et al., Biomaterials. 2002, 23(9):2003-13 in which encapsulation of mesenchymal progenitor cell line in alginate is disclosed; Ponce S., et al., J Control Release. 2006, 116(1):28-34, Abbah et al, BBRC 347, 2006, pages 185-191 and Markusen et al, tissue engineering 12(4), 2006, page 821-830..

### SUMMARY OF THE INVENTION

The method of producing encapsulated primary mesenchymal stem cells of the present invention is defined in claim 1, the composition of matter of the present invention is defined in claim 2, the method of ex-vivo proliferating and/or differentiating mesenchymal stem cells of the present invention is defined in claim 3, the pharmaceutical composition of the present invention is defined in claim 5.

The present invention relates to a method of producing encapsulated primary mesenchymal stem cells comprising:
(a) providing a population of cells which comprise at least 97 % primary mesenchymal stem cells with an alginate to thereby obtain a solution of said alginate with said primary mesenchymal stem cells, and
(b) infusing droplets of said solution into a Calcium Chloride (CaCl₂) solution, to thereby generate spheres of said alginate and said mesenchymal stem cells,
(c) mixing said spheres with a poly-L lysine (PLL) solution, to thereby create semi permeable microcapsules,
thereby producing the encapsulated mesenchymal stem cells.

The present invention also relates to a composition-of-matter comprising an alginate poly-L lysine (PLL) microcapsule encapsulating primary mesenchymal stem cells generated according to the above mentioned method.

The present invention also relates to a method of ex-vivo proliferating and/or differentiating mesenchymal stem cells, comprising culturing the above mentioned composition-of-matter under conditions required for the proliferation and/or differentiation of mesenchymal stem cells, thereby proliferating and/or differentiating mesenchymal stem cells.

Preferably the composition-of-matter is used for transplantation in a subject in need thereof.

The present invention also relates to a pharmaceutical composition comprising the above mentioned composition-of-matter and a pharmaceutically acceptable carrier.

The composition-of-matter, the method, or the pharmaceutical composition of the present invention, have mesenchymal stem cells which do not express a heterologous polynucleotide.

The composition-of-matter of, the method , or the pharmaceutical composition, have mesenchymal stem cells which express a heterologous polynucleotide.

The composition-of-matter, the method, or the pharmaceutical composition of the present invention, having mesenchymal stem cells being derived from a tissue selected from the group consisting of bone marrow, adipose tissue, embryonic yolk sac, placenta, umbilical cord and skin.

The composition-of-matter, the method, or the pharmaceutical composition of the present invention, having mesenchymal stem cells expressing surface markers including CD105, CD90, CD44 and CD29 and not CD31, CD34, CD144 and CD133.

The composition-of-matter, the method, or the pharmaceutical composition of the present invention, having mesenchymal stem cells are capable of differentiating into osteoblasts, chondrocytes and adipocytes.

The composition-of-matter, the method, or the pharmaceutical composition of the present invention, having heterologous polynucleotide comprising a therapeutic polynucleotide.

The composition-of-matter of the present invention, wherein the subject in need thereof has a medical condition selected from the group consisting of stem cell deficiency, heart disease, Parkinson's disease, cancer, Alzheimer's disease, stroke, burns, loss of tissue, loss of blood, anemia, autoimmune disorders, diabetes, arthritis, Multiple Sclerosis, graft versus host disease (GvHD), a neurodegenerative disorder, autoimmune encephalomyelitis (EAE), systemic lupus erythematosus (SLE), rheumatoid arthritis, systemic sclerosis, Sjorgen's syndrome, multiple sclerosis (MS), Myasthenia Gravis (MG), Guillain- Barré Syndrome (GBS), Hashimoto's Thyroiditis (HT), Graves's Disease, Insulin dependent Diabetes Melitus (IDDM) and Inflammatory Bowel Disease.

The composition-of-matter, the method, or the pharmaceutical composition of the present invention, having a primary mesenchymal stem cells maintaining their proliferative capacity within said microcapsule.

The composition-of-matter, the method, or the pharmaceutical composition of the present invention, having a proliferative capacity maintained after 90 days in culture.

### BRIEF DESCRIPTION OF THE DRAWINGS

Some embodiments of the invention are herein described, by way of example only, with reference to the accompanying drawings. With specific reference now to the drawings in detail, it is stressed that the particulars shown are by way of example and for purposes of illustrative discussion of embodiments of the invention. In this regard, the description taken with the drawings makes apparent to those skilled in the art how embodiments of the invention may be practiced.

In the drawings:

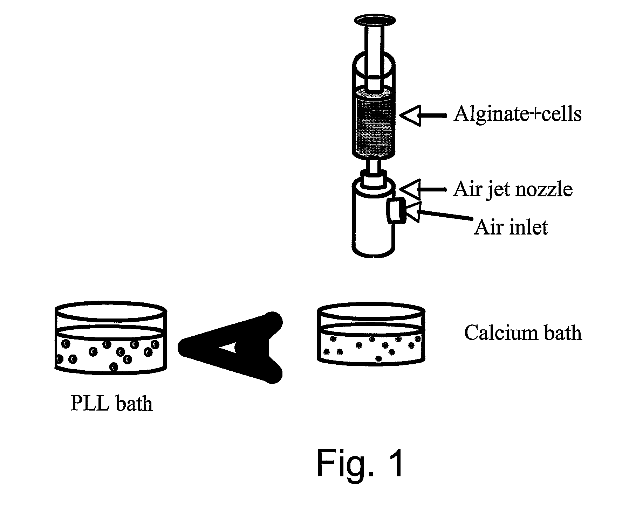

FIG. 1 is a schematic illustration depicting the process of cell encapsulation in alginate-PLL microcapsules. Alginate PLL microcapsules are prepared using the following technique; alginate solution composing the entrapped cells with a known concentration is infused at a constant rate through a narrow nozzle. Constant airflow intersects the alginate cells mixture into droplets. The size and shape of the droplets depend on the nozzle diameter, the alginate solution flow rate, air pressure, the droplets falling distance, and the alginate solution viscosity. The droplets fall into a well-mixed CaCl₂ bath solution. After the hydrogel is formed the spheres are washed, filtered, and mixed with PLL solution to create to final semi permeable microcapsules.

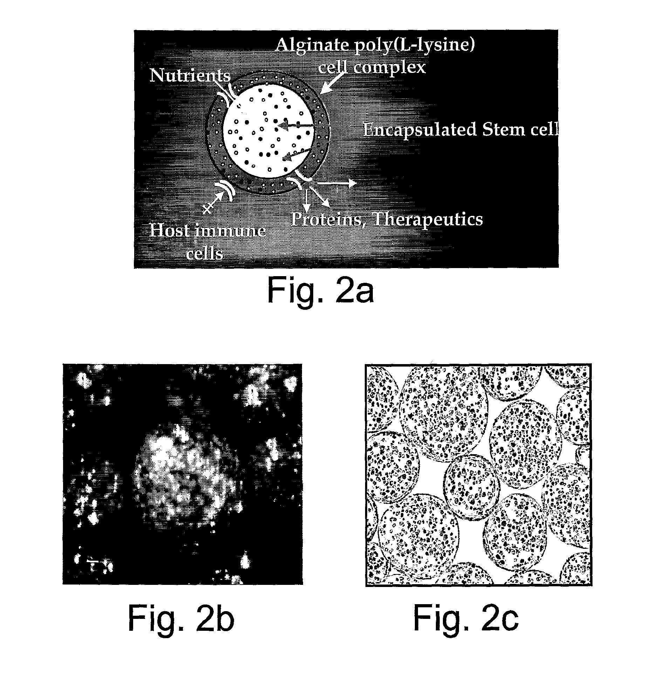

FIGs. 2a-c are a schematic illustration (Figure 2a) and photomicrographs (Figures 2b and c) depicting an exemplary cell encapsulation system according to some embodiments of the invention. Figure 2a - a schematic illustration of the microcapsule structure. The red circle depicts a semi permeable membrane that is formed from alginate (a polysaccharide) and poly (L-lysine) (PLL; a poly amino acid). Figure 2b - a fluorescent micrograph of human mesenchymal stem cells (red) encapsulated in the alginate-PLL capsules. Green fluorescent is PLGA particles entrapping growth factors including VEGF, bFGF and PDGF or anti inflammatory drugs including Ibuprofene. Figure 2c - a light micrograph of human mesenchymal stem cells (the dark dots in the capsules) encapsulated in alginate-PLL microcapsules.

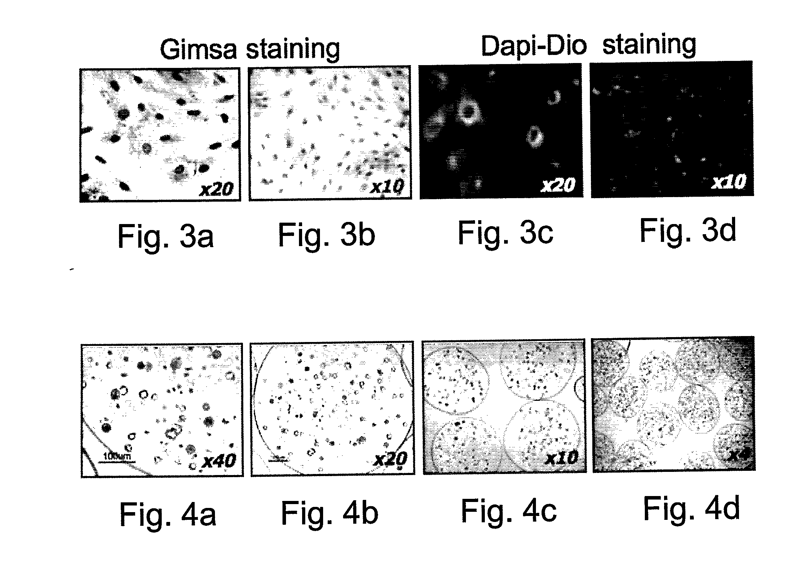

FIGs. 3a-d are photomicrographs of a human mesenchymal stem cells (MSCs) culture stained with Giemsa stain (Figures 3a and b) or Dapi-Dio staing (Figures 3c and d). Note the typical spindle like shape morphology of human MSCs in the alginate-PLL microcapsules.
FIGs. 4a-d are images of phase contrast microscopy of hMSCs-loaded microcapsules, demonstrating the uniform size and cell distribution (average capsule diameter 0.6 mm).

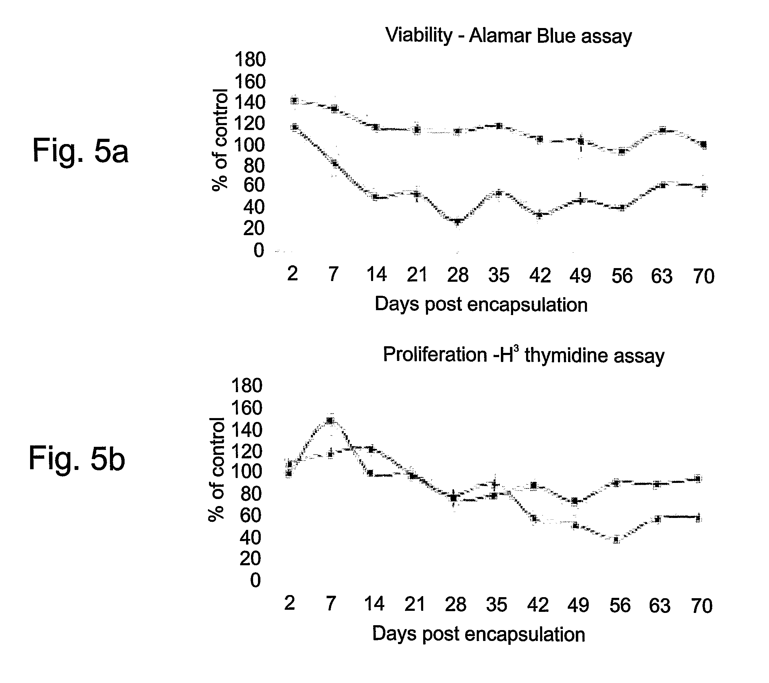

FIGs. 5a-b are graphs which depict viability (Figure 5a) and proliferation (Figure 5b) of human MSCs in capsule (red squares) in comparison to the NIH-3T3 cell line (blue triangles) which is used frequently for encapsulation. Results are presented as percentages of control cells (non-encapsulated cells).

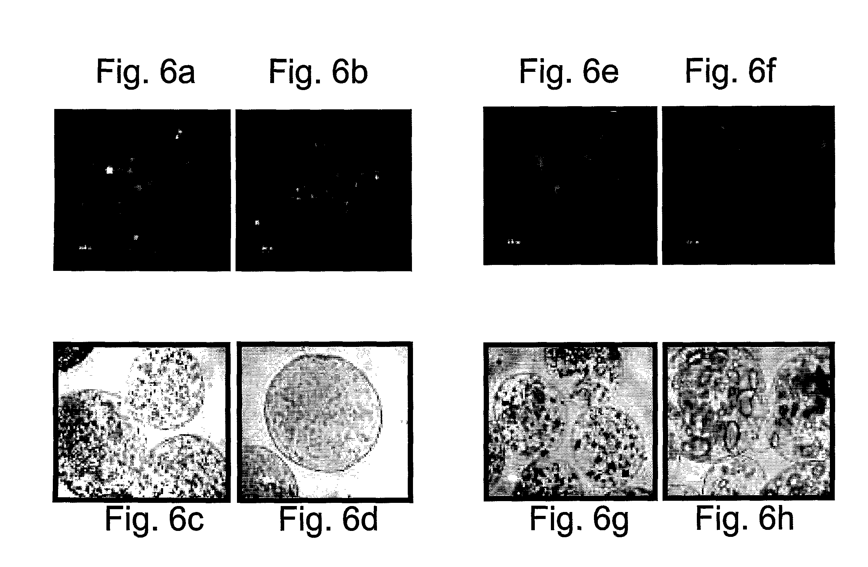

FIGs. 6a-h are micrographs of human MSCs Ps. 3 (Figures 6a-d) or HIH 3T3 cell line (Figures 6e-h) stained with fluorimetric qualitative fluorescein diacetate assay (FDA; green fluorescence) for viability evaluation (Figures 6a, b, e and f) or analysed by phase contrast microscopy (Figures 6c, d, g and h) at 28 (Figures 6a, c, e and g) or 70 (Figures 6b, d, f and h) days after encapsulation in alginate-PLL microcapsules.

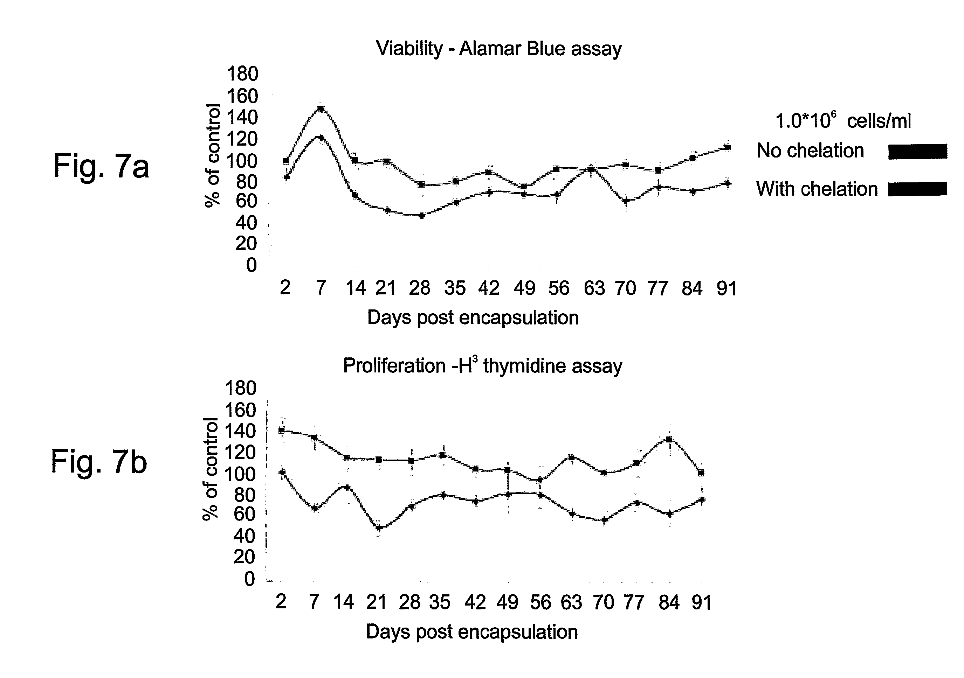

FIGs. 7a-b are graphs depicting viability (Figure 7a) and proliferation (Figure 7b) of MSCs in alginate-PLL microcapsules which were either treated with Calcium chelation (blue plots) or remained untreated (red plots).

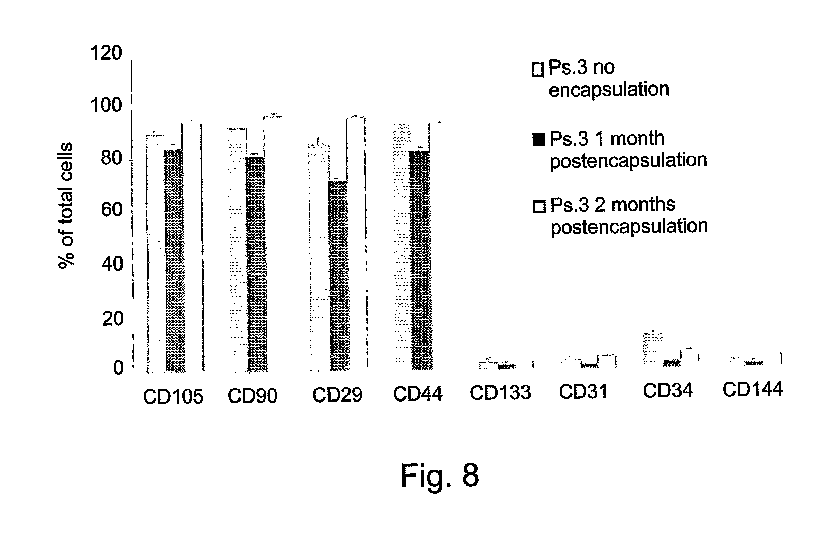

FIG. 8 is a histogram demonstrating results of FACS analyses peprformed on MSCs Ps.3 using antibodies directed against: CD105, CD90, CD29, CD44, CD133, CD31, CD34 and CD144. Non-encapsulated (blue) and encapsulated hMSCs which were retrieved 1 (red column) and 2 (green column) months post encapsulation were characterized for surface marker analysis using flow cytometry assay. Note that MSCs maintain their phenotype and morphology in capsules (one and two months after encapsulation).

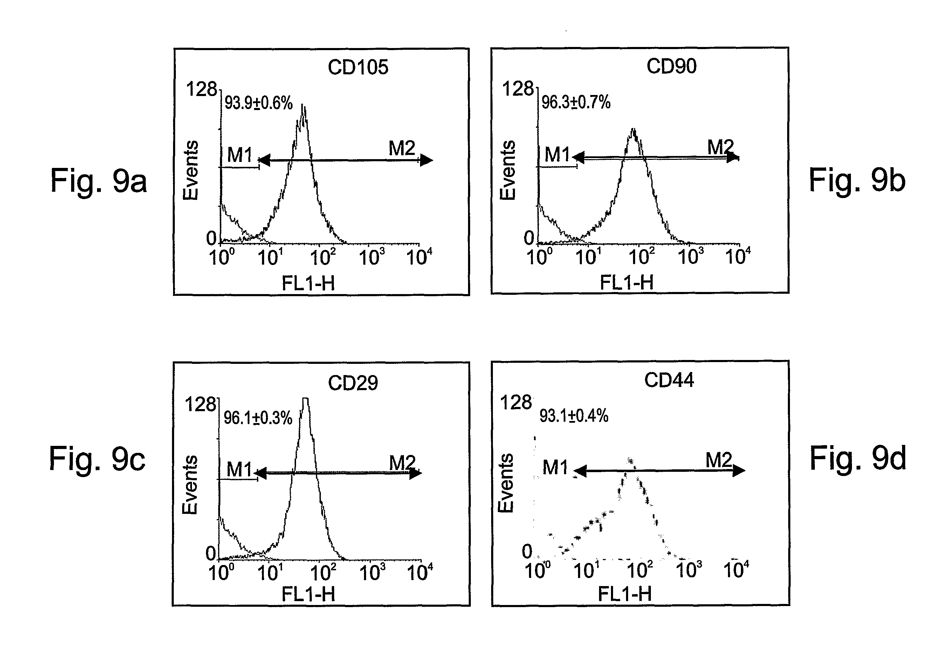

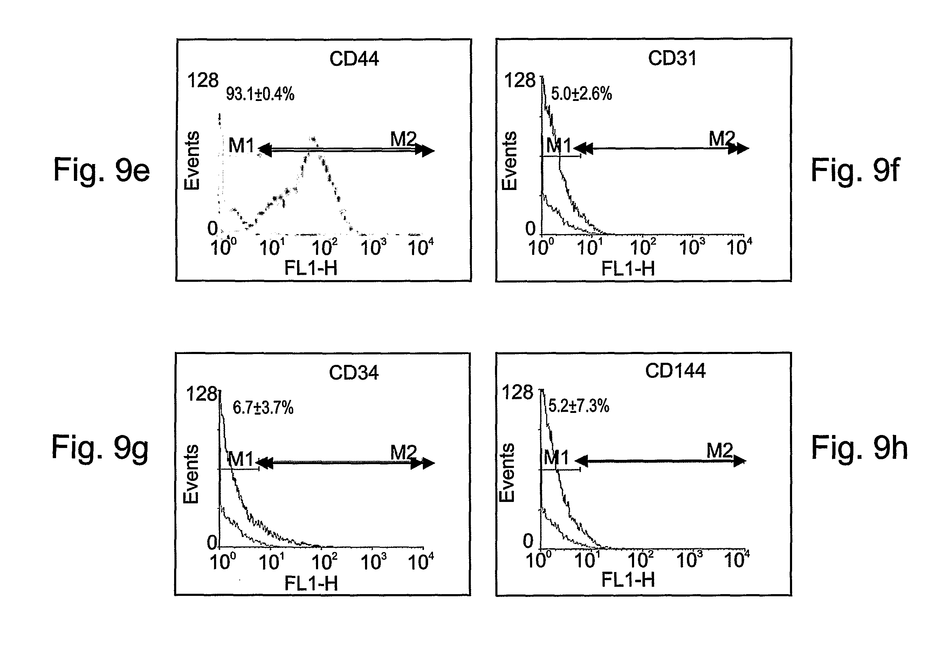

FIGs. 9a-h are FACS analyses of hMSCs performed on retrieved encapsulated hMSCs two months post encapsulation using antibodies directed against: CD105 (Figure 9a), CD90 (Figure 9b), CD29 (Figure 9c), CD44 (Figure 9d), CD133 (Figure 9e), CD31 (Figure 9f), CD34 (Figure 9g) and CD144 (Figure 9h).

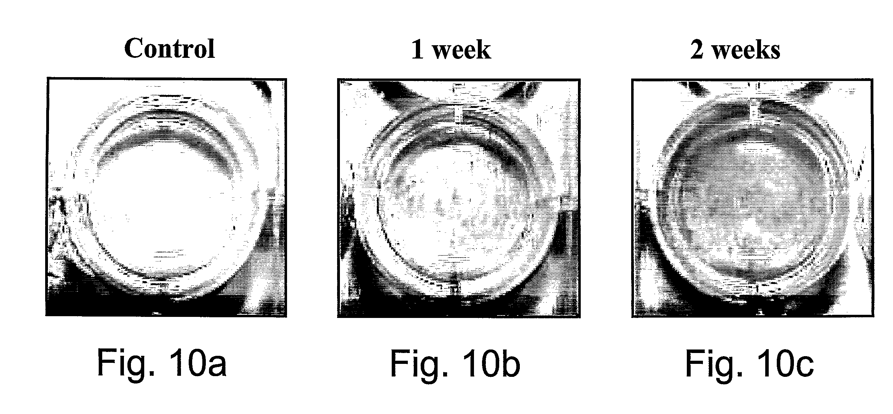

FIGs. 10a-c are images of encapsulated hMSCs stained with van kossa staining (free calium staining). Encapsulated hMSCs were cultured in the osteogenic induction medium and following one or two weeks were stained for presence of free clacium, a marker of obsteoblast differentiation. Figure 10a - control (MSCs in an undifferentiated buffer) Figure 10b - hMSCs following one week in the osteoblast differentiation medium; Figure 10c - hMSCs following two weeks in the osteoblast differentiation medium.

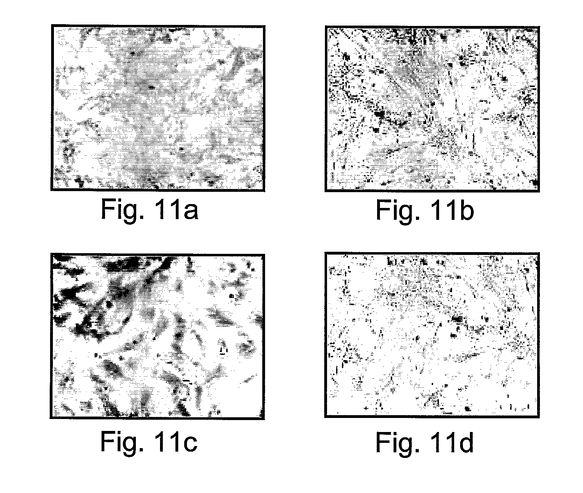

FIGs. 11a-d are images of retrieved hMSCs which were differentiated in the alginate-PLL microcapsules into osteoblasts or remained undifferentiated. Figure 11a - hMSCs retrieved from the microcapsules following 2 weeks in osteoblast differentiation medium stained with alkaline phosphatase; Figure 11b - non-differentiated hMSCs retrieved from the microcapsules stained with alkaline phosphatase; Figure 11c - hMSCs retrieved from the microcapsules following 2 weeks in osteoblast differentiation medium and stained with van kossa staining; Figure 11d - non-differentiated hMSCs retrieved from the microcapsules and stained with van kossa staining;

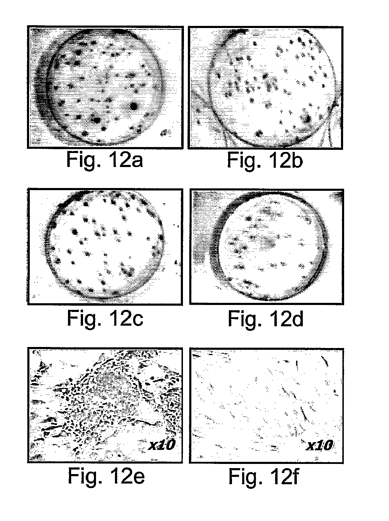

FIGs. 12a-f are images of encapsulated hMSCs which were differentiated in the alginate-PLL microcapsules into adipocyte, chondrocytes or remianed undifferentiated. Figure 12a - hMSCs in the microcapsules following 3 weeks in the adipocyte differentiation medium stained with Oil red O staining; Figure 12b - non-differentiated hMSCs in the microcapsules stained with Oil red O staining; Figure 12c - hMSCs in the microcapsules following 3 weeks in the chondrocyte differentiation medium stained with Alcian Blue staining; Figure 12d - non-differentiated hMSCs in the microcapsules stained with Alcian Blue staining; Figure 12e - hMSCs retreived from the microcapsules following 3 weeks in the chondrocyte differentiation medium and stained with Alcian Blue staining; Figure 12f - non-differentiated hMSCs retrieved from the microcapsules and stained with Alcian Blue staining;

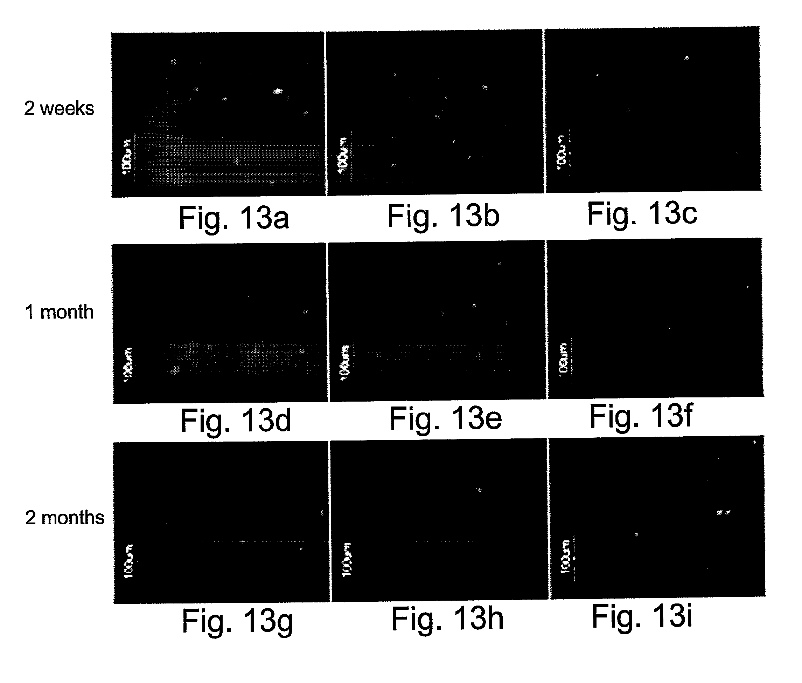

FIGs. 13a-i are fluorescent micrscope images of encapsulated MSCs demonstrating expression of green fluorescent protein. MSCs were transfected with pEGF and encapsulated in microcapsules. Shown are the transfected MSCs after two weeks (Figures 13a-c), one month (Figures 13d-f) or two months (Figures 13g-i) of the transfection. Note that encapsulated MSCs maintain their transfection over 2 months.

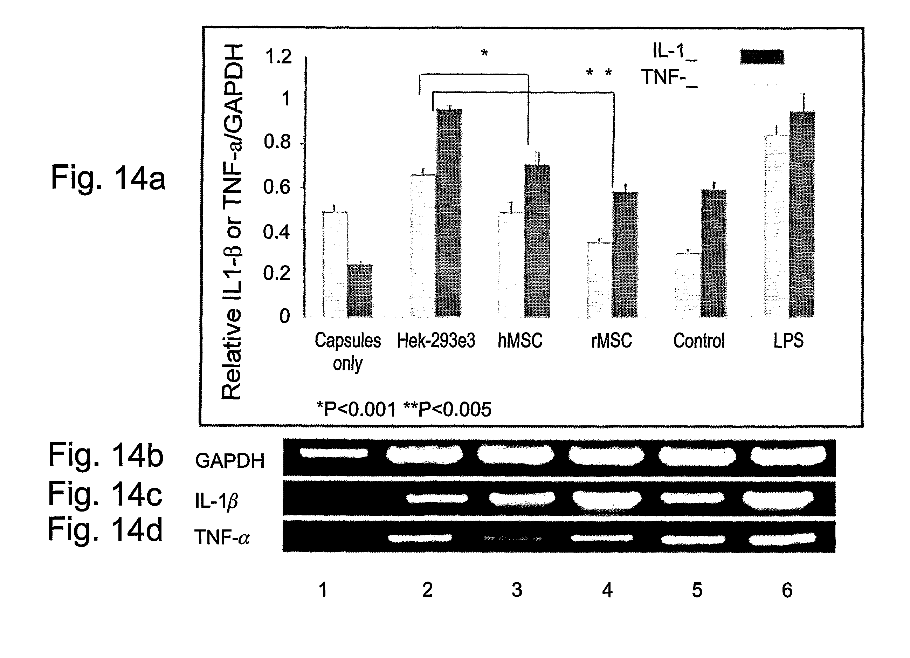

FIGs. 14a-d are a histogram (Figure 14a) and images of gel electrophoresis (Figures 14b-d) depicting RT-PCR analyses of interleukin 1-β (Figure 14c; and purple bars in Figure 14a) or TNF-α (Figure 14d; and blue bars in Figure 12a) levels after the addition of empty capsules or various encapsulated cells to the spleen cells ex vivo. after 48hrs the spleen cells were harvested and lysed and RNA was purified. Note that encapsulated MSCs are less immunogenic, e.g., stimulating less IL-1-β or TNF-α, than HEK 293 cells and are as the control the control is medium only, LPS (Lipo poly saccharin) is used to stimulate the spleen cells as a control group.

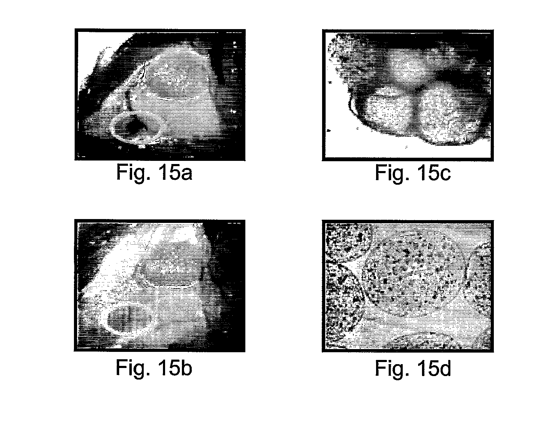

FIGs. 15a-b are micrographs of the right inguinal lymph nodes and of the microcapsules cluster in site of transplantation. Figure 15a - hek-293 microcapsules; Figure 15b - hMSCs microcapsules.
FIGs. 15c-d are images of microcapsules retrieved after the transplantation (as shown in Figures 15a-b) demonstrating cell overgrowth. Figure 15c - hek-293 microcapsules; Figure 15d - hMSCs microcapsules.

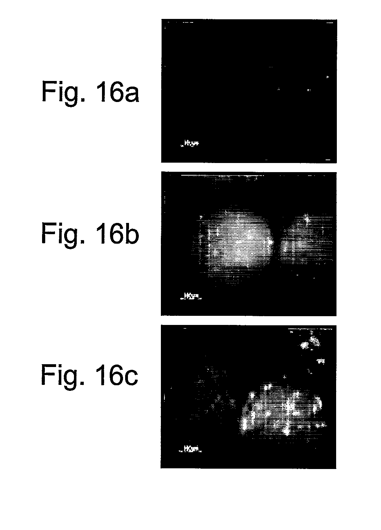

FIGs. 16a-c are images of microcapsules retrieved after the transplantation (as shown in Figures 15a-b) stained with FDA. Figure 16a - rat MSCs (rMSCs) microcapsules; Figure 16b - hMSCs microcapsules; Figure 16c - hek-293 microcapsules.

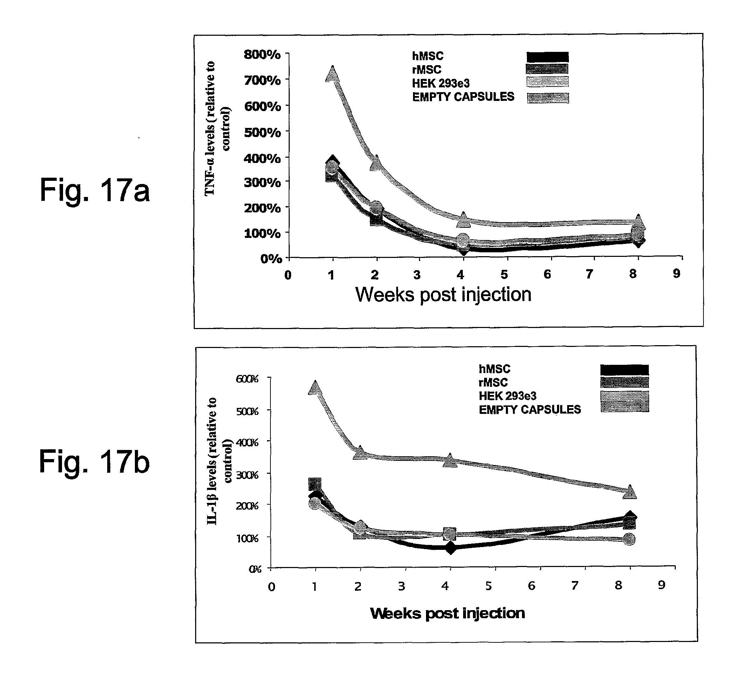

FIGs. 17a-b are graphs depicting the expression level of inflammatory cytokines: TNF-α (Figure 17a) and IL-1β (Figure 17b) in RNA samples of inguinal lymph nodes as determined using RT-PCR analysis. Note that higher levels of TNF-α and IL-1β were observed in the hek-293 group as compared to the MSCs groups.

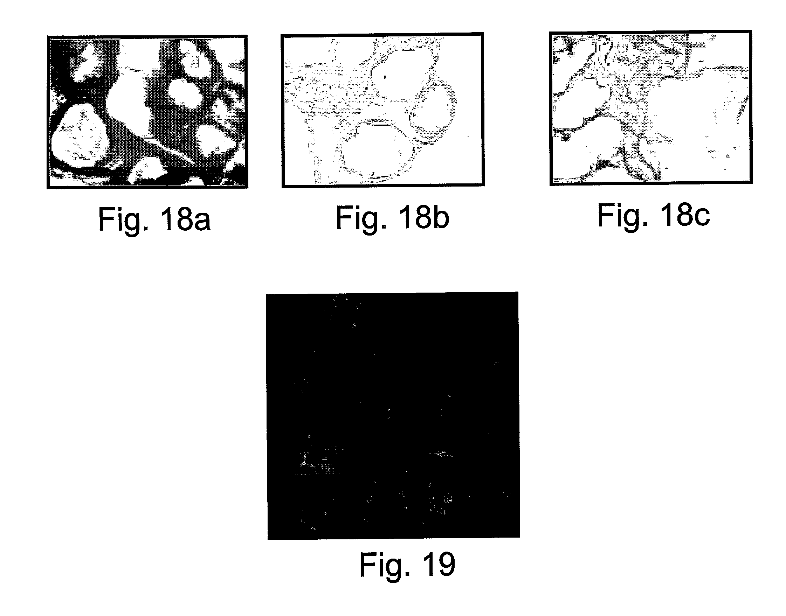

FIGs. 18a-c are images of histological analyses (by H&E staining) of the tissue surrounding the microcapsules after retrieval of the transplanted microcapsules. Figure 18a - microcapsules containing hek-293 cells; Figure 18b - empty microcapsules; Figure 18c - microcapsules containing hMSCs. Note that the encapsulated hek-293 cells were covered with a thick layer of host cells, while in the encapsulated MSCs this was not evident.
FIG. 19 is a microscopical image of hMSCs demonstrating expression of the m-Cherry marker in lentivirus transduced hMSCs.

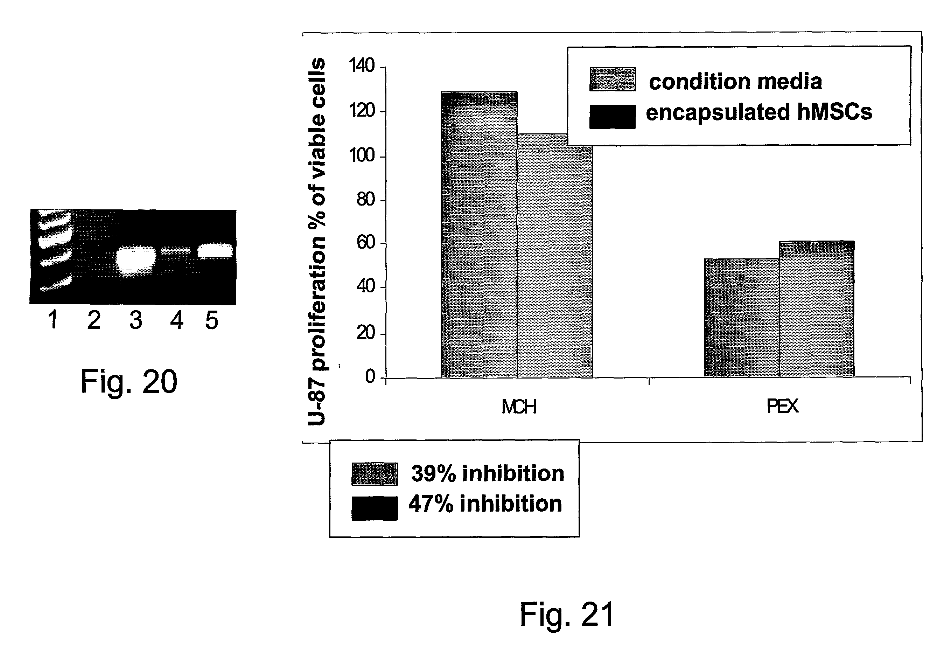

FIG. 20 is an image of agarose gel depicting PEX DNA or RNA PCR analysis in hMSCs. Lane 1 - DNA marker; lane 2 - hMSCs transduced with an empty vector; lane 3 - PEX positive control; lane 4 - DNA encoding PEX in the PEX transduced hMSCs; lane 5 - RNA encoding exogenous PEX in the PEX transduced hMSCs. Note the presence of PEX DNA (lane 4) and PEX RNA (lane 5) in hMSCs transduced with the PEX DNA and the absence of PEX DNA in hMSCs transduced with an empty vector.
FIG. 21 is a histogram depicting inhibition of U-87 cell proliferation by PEX-expressing encapsulated hMSCs. 20,000 U-87 cells were grown in transwells. After 24 hrs condition media from PEX expressing encapsulated hMSCs was collected and added to the wells or the encapsulated hMSCs were placed on inserts; proliferation was measured by H³ thyimidine uptake assay. Note that PEX-expressing encapsulated hMSCs inhibited U-87 cells proliferation by 47 %.

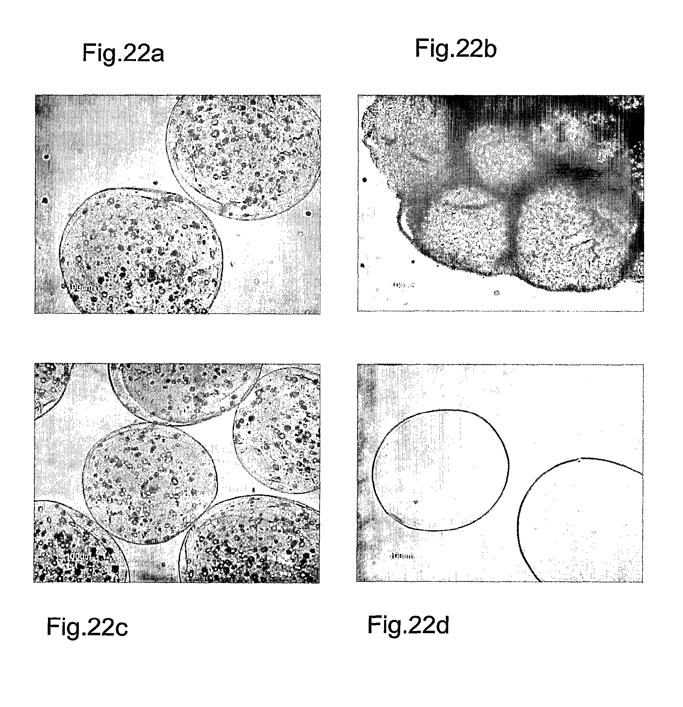

FIGs. 22a-d are images of capsules containing human MSCs (Figure 22a), rat MSCs (Figure 22c), or HEK 293 cell line (Figure 22b) or empty capsules (Figure 22d) two months post transplantation in mice. The microcapsules were retrieved from the transplanted animals and photographed. Note that the capsule with HEK 293 cell line led to a fibrotic reaction around the capsule (Figure 22b) which demonstrate the immunological stimulation of these cells compared to the non-immunological reaction in the retrieved human (Figure 22a) or rat (Figure 22c) MSCs.

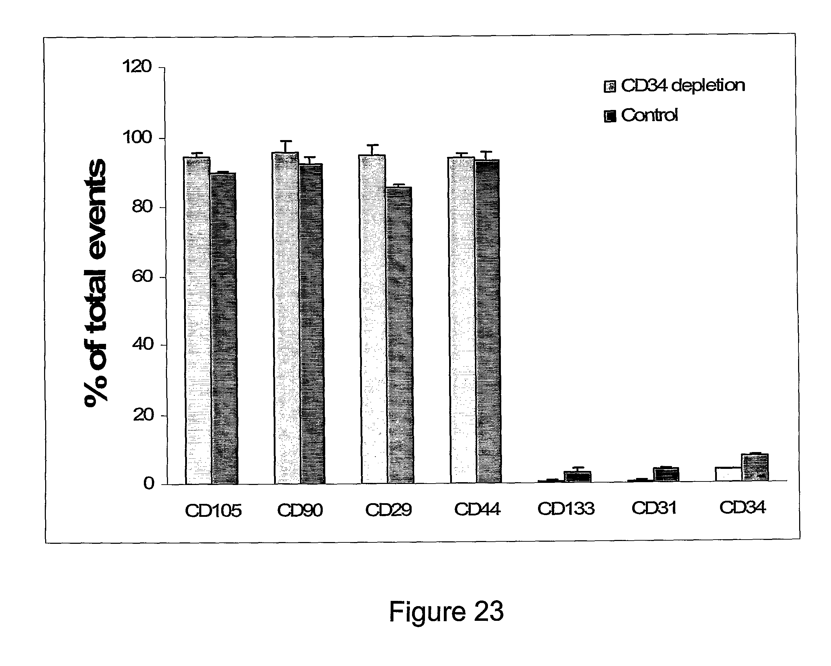

FIG. 23 is an immuno-depletion of bone marrow sample effected to omit hematopoietic cells (CD34+). Briefly, hMSCs were passed through MACS colony (mouse anti human CD34 555820, and CD31 555444, Becton Dickson) several times and then washed and analyzed by phenotypic marker characterization using FACS. The results showed resemblance in phenotypic characterization of hMSCs. Immunodepletion reduced CD34+ cells to less than 2% in the sample.

### DESCRIPTION OF SPECIFIC EMBODIMENTS OF THE INVENTION

The present invention, in some embodiments thereof, relates to encapsulated mesenchymal stem cells and their use in tissue engineering and therapy.

Before explaining at least one embodiment of the invention in detail, it is to be understood that the invention is not necessarily limited in its application to the details set forth in the following description or exemplified by the Examples. The invention is capable of other embodiments or of being practiced or carried out in various ways.

Entrapment of mammalian cells in physical membranes physically isolates a cell mass from an outside environment and aims to maintain normal cellular physiology within a desired permeability barrier. Numerous encapsulation techniques have been developed to date. However, despite considerable interest, the field of cell encapsulation has not lived up to expectations. In the case of stem cell encapsulation maintaining stem cell viability and differentiation potency has been proven especially difficult.

Whilst reducing the present invention to practice the present inventors were able, for the first time, to encapsulate mesenchymal stem cells (MSCs) while maintaining their proliferative capacity and differentiation potential for an unprecedented period of time. Compositions comprising these encapsulated cells can be used in a myriad of clinical, tissue engineering and research applications.

As is illustrated hereinbelow and in the Examples section which follows, the present inventors realized that in order to efficiently encapsulate MSCs while maintaining their biological functions (e.g., self-renewal and multipotency), a highly purified (i.e., above 97 %) pupolation of MSCs must be used. Bone marrow aspirates were seeded on plates, adherent cells collected and using magnetic antibody capture protocol, highly purified populations comprising at least about 97 % MSCs were collected. These cells were subject to encapsulation using a biocompatible, chemically and mechanically stable membrane composed of alginate and PLL. The encapsulation procedure is illustrated in Figure 1. Encapsulated cells were shown to maintain MSC morphology, viability and proliferation capacity even after 90 days in culture (see Figures 3a-d - Figure 8). The cells were shown to maintain their multipotency as evidenced by their ability to differentiate into osteoblasts (Figures 10a-c and 11a-d), chondrocytes and adipocytes (Figure 12a-f). Encapsulated cells were shown biocompatible as evidenced by the minimal immune response following transplantation (production of pro-inflammatory cytokines, Figures 12a-f to 16a-c). The present inventors have also shown that naïve and genetically engineered cells can be used in accordance with the present teachings, as shown in Figures 19-21.

All these findings point out that the present teachings provide biocompatible encapsulated MSCs which are biologically functional for unprecedented time *in vivo.* These encapsulated cells can be genetically engineered to improve their *in vivo* viability and alternatively or additionally to continuously produce therapeutic products (e.g., neurotransmitters, see further below).

Thus, according to an aspect of the present invention there is provided a method of producing encapsulated mesenchymal stem cells. The method comprising providing a population of cells which comprise at least 97 % mesenchymal stem cells, and encapsulating said population of cells in a microcapsule, thereby producing the encapsulated mesenchymal stem cells.

As used herein the phrase "mesenchymal stem cells" (MSCs) refers to fetal or postnatal (e.g., adult) cells which differentiate (either terminally or non-terminally) to give rise to cells of a mesenchymal and under certain conditions mesodermal cell lineage and which are also capable of dividing to yield stem cells. The cells can be primary cells or derived from mesenchymal stem cell lines.

Thus, mesenchymal stem cells give rise to one or more mesenchymal tissues (e.g., adipose, osseous, cartilaginous, elastic and fibrous connective tissues, myoblasts) as well as to tissues other than those originating in the embryonic mesoderm (e.g., neural cells) depending upon various influences from bioactive factors such as cytokines. Cells differentiated into any of these lineages are envisaged by the present teachings.

Mesenchymal stem cells are also referred to as marrow stromal cells or multipotent stromal cells.

MSCs according to the present teachings are adherent cells which express the surface markers CD105, CD90, CD44 and CD29 and which do not express the CD34, CD 31, CD 144 and CD133 surface markers.

The mesenchymal stem cells of the present invention may be of a xenogeneic or allogeneic source.

Mesenchymal stem cells of the present invention can be obtained from a plurality of tissues including bone marrow, embryonic yolk sac, placenta, umbilical cord, fetal and adolescent skin, peripheral blood and other tissues. However, their abundance in the BM far exceeds their abundance in other tissues and as such isolation from BM is presently preferred.

A method of isolating mesenchymal stem cells from peripheral blood is described by Kassis et al [Bone Marrow Transplant. 2006 May;37(10):967-76]. A method of isolating mesenchymal stem cells from placental tissue is described by Zhang et al [Chinese Medical Journal, 2004, 117 (6):882-887]. Methods of isolating and culturing adipose tissue, placental and cord blood mesenchymal stem cells are described by Kern et al [Stem Cells, 2006;24:1294-1301].

According to an embodiment of this aspect of the present invention, the mesenchymal stem cells are isolated from humans.

MSCs are typically first plated on adherent (e.g., polystyrene plastic) surfaces (e.g. in a flask) and mesenchymal stem cells are isolated by removing non-adherent cells. Thereafter, mesenchymal stem cell are further purified using methods which are well known in the art such as antibody-based techniques, e.g., FACS or MAC using mesenchymal stem cell markers (positive and/or negative selection), as described in details in the Examples section which follows.

Although the preparation of BM-derived MSCs is described in details infra, isolation from other tissues (as mentioned above) is to be regarded as falling under the scope of the present invention. See the Examples section for a detailed protocol of isolation and purification.

An alternative protocol is provided as follows. A bone marrow aspirate from the iliac crest of an individual is diluted (usually 20 ml) with equal volumes of Hank's balanced salt solution (HBSS; GIBCO Laboratories, Grand Island, NY, USA) and layered over about 10 ml of a Ficoll column (Ficoll-Paque; Pharmacia, Piscataway, NJ, USA). Following 30 minutes of centrifugation at 2,500 x g, the mononuclear cell layer is removed from the interface and suspended in HBSS. Cells are then centrifuged at 1,500 x g for 15 minutes and resuspended in a complete medium (MEM, α medium without deoxyribonucleotides or ribonucleotides; GIBCO); 20 % fetal calf serum (FCS) derived from a lot selected for rapid growth of MSCs (Atlanta Biologicals, Norcross, GA); 100 units/ml penicillin (GIBCO), 100 µg/ml streptomycin (GIBCO); and 2 mM L-glutamine (GIBCO). Resuspended cells are plated in about 25 ml of medium in a 10 cm culture dish (Corning Glass Works, Corning, NY) and incubated at 37 °C with 5 % humidified CO₂. Following 24 hours in culture, nonadherent cells are discarded, and the adherent cells are thoroughly washed twice with phosphate buffered saline (PBS). Adherent cells may be immediately purified or further cultured and then purified. Thus for further culturing of adherent cells, the medium is replaced with a fresh complete medium every 3 or 4 days for about 14 days. Adherent cells are then harvested with 0.25 % trypsin and 1 mM EDTA (Trypsin/EDTA, GIBCO) for 5 min at 37 °C, replated in a 6-cm plate and cultured for another 14 days. Cells are then trypsinized and counted using a cell counting device such as for example, a hemocytometer (Hausser Scientific, Horsham, PA). Cultured cells are recovered by centrifugation and resuspended with 5 % DMSO and 30 % FCS at a concentration of 1 to 2 X 10⁶ cells per ml. Aliquots of about 1 ml each are slowly frozen and stored in liquid nitrogen.

To expand the mesenchymal stem cell fraction, frozen cells are thawed at 37 °C, diluted with a complete medium and recovered by centrifugation to remove the DMSO. Cells are resuspended in a complete medium and plated at a concentration of about 5,000 cells/cm². Following 24 hours in culture, nonadherent cells are removed and the adherent cells are harvested using Trypsin/EDTA, dissociated by passage through a narrowed Pasteur pipette, and preferably replated at a density of about 1.5 to about 3.0 cells/cm². Under these conditions, MSC cultures can grow for about 50 population doublings and be expanded for about 2000 fold [Colter DC., et al. Rapid expansion of recycling stem cells in cultures of plastic-adherent cells from human bone marrow. Proc Natl Acad Sci USA. 97: 3213-3218, 2000].

Purification is effected by positive selection using at least the following surface markers CD105, CD90, CD44 and CD29 and negatively selected against CD34, CD 31, CD144 and CD133 surface markers. Antibodies for any of the above markers are commercially available such as described in the Examples section which follows.

Qualification and purity can be determined using methods which are well known in the art including morphology assays (described in the examples section), as well as molecular methods assaying marker expression at the mRNA (RT-PCR) or protein level (immunoassays).

Once purified MSC populations are obtained they are encapsulated using methods and compositions which are well known in the art.

The primary goal in encapsulation as a cell therapy is to protect allogeneic and xenogeneic cell transplants from destruction by the host immune system, thereby eliminating or reducing the need for immuno-suppressive drug therapy. Techniques for microencapsulation of cells are known to those of skill in the art (see, for example, Chang, P. et al. 1999; Matthew, H. W. et al. 1991; Yanagi, K. et al. 1989; Cai Z. H. et al. 1988; Chang, T. M. 1992).

Encapsulation techniques are generally classified as microencapsulation, involving small spherical vehicles and macroencapsulation, involving larger flat-sheet and hollow-fiber membranes (Uludag, H. et al. Technology of mammalian cell encapsulation. Adv Drug Deliv Rev. 2000; 42: 29-64).

Methods of preparing microcapsules are known in the arts and include for example those disclosed by Lu MZ, et al., Cell encapsulation with alginate and alpha-phenoxycinnamylidene-acetylated poly(allylamine). Biotechnol Bioeng. 2000, 70: 479-83, Chang TM and Prakash S. Procedures for microencapsulation of enzymes, cells and genetically engineered microorganisms. Mol Biotechnol. 2001, 17: 249-60, and Lu MZ, et al., A novel cell encapsulation method using photosensitive poly(allylamine alpha-cyanocinnamylideneacetate). J Microencapsul. 2000, 17: 245-51.

For example, microcapsules are prepared by complexing modified collagen with a ter-polymer shell of 2-hydroxyethyl methylacrylate (HEMA), methacrylic acid (MAA) and methyl methacrylate (MMA), resulting in a capsule thickness of 2-5 µm. Such microcapsules can be further encapsulated with additional 2-5 µm ter-polymer shells in order to impart a negatively charged smooth surface and to minimize plasma protein absorption (Chia, S.M. et al. Multi-layered microcapsules for cell encapsulation Biomaterials. 2002 23: 849-56).

Other microcapsules are based on alginate, a marine polysaccharide (Sambanis, A. Encapsulated islets in diabetes treatment. Diabetes Thechnol. Ther. 2003, 5: 665-8) or its derivatives. For example, microcapsules can be prepared by the polyelectrolyte complexation between the polyanions sodium alginate and sodium cellulose sulphate with the polycation poly(methylene-co-guanidine) hydrochloride in the presence of calcium chloride.

It will be appreciated that cell encapsulation is improved when smaller capsules are used. Thus, the quality control, mechanical stability, diffusion properties, and *in vitro* activities of encapsulated cells improved when the capsule size was reduced from 1 mm to 400 µm (Canaple L. et al., Improving cell encapsulation through size control. J Biomater Sci Polym Ed. 2002; 13: 783-96). Moreover, nanoporous biocapsules with well-controlled pore size as small as 7 nm, tailored surface chemistries and precise microarchitectures were found to successfully immunoisolate microenvironments for cells (Williams D. Small is beautiful: microparticle and nanoparticle technology in medical devices. Med Device Technol. 1999, 10: 6-9; Desai, T.A. Microfabrication technology for pancreatic cell encapsulation. Expert Opin Biol Ther. 2002, 2: 633-46).

A specific composition and method for of cell encapsulation is described at length in the Examples section which follows. More specifically an alginate-PLL composition is used in which the content of the first is about 1.2 % and the latter (PLL) is 0.06 %.

Other methods of cell encapsulation are well known in the art, such as those described in European Patent Publication No. 301,777 or U.S. Pat. Nos. 4,353,888; 4,744,933; 4,749,620; 4,814,274; 5,084,350; 5,089,272; 5,578,442; 5,639,275; and 5,676,943,. Other methods are described U.S. Pat. 6,281,341,; Desai 2002 Exp. Opin. Biol. Hortelano et al. 1996 Blood 87:5095-5103; Pelegrin et al. 1998 Gene Ther. 5:828-834; Lohr et al. 2001 Lancet 357:1591-1592; Cirone et al. Hum. Gene Ther. 13: 1157-1166.

The ordinary skilled artisan will select a biocompatible, as well as a mechanically and chemically stable membrane of a suitable permeability cut-off value that provides immune protection to the inplant, functional performance, biosafety and long term survival of the graft.

Encapsulated cells generated according to the present teachings can be used in a myriad or research and clinical applications.

Thus, according to another aspect of the present invention there is provided a method of transplanting mesenchymal stem cells such as for treating a medical condition (e.g., pathology, disease, syndrome) which may benefit from stromal stem cell transplantation in a subject in need thereof.

As used herein the term "treating" refers to inhibiting or arresting the development of a pathology and/or causing the reduction, remission, or regression of a pathology. Those of skill in the art will understand that various methodologies and assays can be used to assess the development of a pathology, and similarly, various methodologies and assays may be used to assess the reduction, remission or regression of a pathology. Preferably, the term "treating" refers to alleviating or diminishing a symptom associated with a cancerous disease. Preferably, treating cures, e.g., substantially eliminates, the symptoms associated with the medical condition.

As used herein "a medical condition which may benefit from mesenchymal stem cell transplantation" refers to any medical condition which may be alleviated by administration of the encapsulated cells of the present invention.

Examples of such medical conditions include, but are not limited to, stem cell deficiency, heart disease, neurodegenerative diseases, glaucoma neuropathy, Parkinson's disease, cancer, Schizophrenia, Alzheimer's disease, stroke, burns, loss of tissue, loss of blood, anemia, autoimmune disorders, diabetes, arthritis, Multiple Sclerosis, graft vs. host disease (GvHD), neurodegenerative disorders, chronic pain, autoimmune encephalomyelitis (EAE), systemic lupus erythematosus (SLE), rheumatoid arthritis, systemic sclerosis, Sjorgen's syndrome, multiple sclerosis (MS), Myasthenia Gravis (MG), Guillain- Barré Syndrome (GBS), Hashimoto's Thyroiditis (HT), Graves's Disease, Insulin dependent Diabetes Melitus (IDDM) and Inflammatory Bowel Disease.

Since MSC interfere with dendritic cell and T-cell function and generate a local immunosuppresive microenvironment by secreting cytokines, cells of the present invention may also be used for inhibiting inflammation (and autoimmune diseases as mentioned above). It has also been shown that the immuno-modulatory function of human MSC is enhanced when the cells are exposed to an inflammatory environment characterised by the presence of elevated local interferon-gamma levels.

The term or phrase "transplantation", "cell replacement", "implantation" or "grafting" are used interchangeably herein and refer to the introduction of the cells of the present invention to target tissue.

As used herein the term "subject" refers to any subject (e.g., mammal), preferably a human subject.

The method of this aspect of the present invention comprises administering to the subject a therapeutically effective amount of the encapsulated cells the present invention (described hereinabove), thereby treating the medical condition which may benefit from mesenchymal stem cell transplantation in the subject

The administered cells may be non-differentiated or cells which have been differentiated to any mesenchymal or mesodermal lineage as described hereinabove. Methods of deriving lineage specific cells from the mesenchymal stem cells of the present invention are well known in the art. See for example, U.S. Pat. Nos. 5,486,359, 5,942,225, 5,736,396, 5,908,784 and 5,902,741.

The cells may be naïve (non-genetically modified) or genetically modified such as to derive a lineage of interest (see U.S. Pat. Appl. No. 20030219423) or to promote *in vivo* longevity (AM, adrenomedullin, Jun-Ichiro et al. Tissue Eng. 2006) or to promote neurotransmitter release (e.g., such as by transfecting with tyrosine hydroxylase). Other examples include the transfection of MSCs with preproenkephalin (hPPE), a precursor protein for enkephalin opioid peptides for the treatment of pain (e.g., in terminal cancer patients, Ikuko et al. Cell Transplantatiom 2006 15:225-330). Additional examples include transforming the cells with PEX, VEGF, bFGF, S-trail and/or Endostatin.

Other examples of exogenous polynucleotides which may be expressed in accordance with the present teachings include, but are not limited to, polypeptides such as peptide hormones, antibodies or antibody fragments (e.g., Fab), enzymes and structural proteins or dsRNA, antisense/ribozyme transcripts which can be directed at specific target sequences (e.g., transcripts of tumor associated genes) to thereby downregulate activity thereof and exert a therapeutic effect. Similarly, protective protein antigens for vaccination (see, for example, Babiuk S et al J Control Release 2000;66:199-214) and enzymes such as fibrinolysin for treatment of ischemic damage (US Pat No. 5,078,995 to Hunter et al) may expressed in the stinging cells for transdermal or transcutaneous delivery. The therapeutic agent can also be a prodrug.

Methods of expressing exogenous polynucleotides in mesenchymal stem cells are well known in the art.

As used herein, the term "expressed" when used in context with the exogenous polynucleotide refers to generation of a polynucleotide (transcript) or a polypeptide product.

An integrative or episomal nucleic acid expression construct may be employed.

Thus, the expression construct can be designed as a gene knock-in construct in which case it will lead to genomic integration of construct sequences, or it can be designed as an episomal expression vector.

In any case, the expression construct can be generated using standard ligation and restriction techniques, which are well known in the art (see Maniatis et al., in: Molecular Cloning: A Laboratory Manual, Cold Spring Harbor Laboratory, New York, 1982). Isolated plasmids, DNA sequences, or synthesized oligonucleotides are cleaved, tailored, and religated in the form desired.

At its minimum, the expression vector of the present invention comprises a polynucleotide encoding the gene of interest (e.g., SMAD8).

The expression vector of the present invention may also include additional sequences which render this vector suitable for replication and integration in prokaryotes, eukaryotes, or preferably both (e.g., shuttle vectors) and ultimately in the mesenchymal stem cells. Typical cloning vectors contain transcription and translation initiation sequences (e.g., promoters, enhancers) and transcription and translation terminators (e.g., polyadenylation signals).

In addition to the elements already described, the expression vector of the present invention may contain other specialized elements intended to increase the level of expression of cloned nucleic acids or to facilitate the identification of cells that carry the recombinant DNA. For example, a number of animal viruses contain DNA sequences that promote the extra chromosomal replication of the viral genome in permissive cell types. Plasmids bearing these viral replicons are replicated episomally as long as the appropriate factors are provided by genes either carried on the plasmid or with the genome of the host cell.

The vector may or may not include a eukaryotic replicon. If a eukaryotic replicon is present, then the vector is amplifiable in eukaryotic cells using the appropriate selectable marker. If the vector does not comprise a eukaryotic replicon, no episomal amplification is possible. Instead, the recombinant DNA integrates into the genome of the engineered cell, where the promoter directs expression of the desired nucleic acid.

Examples of mammalian expression vectors include, but are not limited to, pcDNA3, pcDNA3.1(+/-), pGL3, pZeoSV2(+/-), pSecTag2, pDisplay, pEF/myc/cyto, pCMV/myc/cyto, pCR3.1, pSinRep5, DH26S, DHBB, pNMT1, pNMT41, pNMT81, which are available from Invitrogen, pCI which is available from Promega, pMbac, pPbac, pBK-RSV and pBK-CMV which are available from Strategene, pTRES which is available from Clontech, and their derivatives.

Expression vectors containing regulatory elements from eukaryotic viruses such as retroviruses can be also used. SV40 vectors include pSVT7 and pMT2. Vectors derived from bovine papilloma virus include pBV-1MTHA, and vectors derived from Epstein Bar virus include pHEBO, and p2O5. Other exemplary vectors include pMSG, pAV009/A⁺, pMTO10/A⁺, pMAMneo-5, baculovirus pDSVE, and any other vector allowing expression of proteins under the direction of the SV-40 early promoter, SV-40 later promoter, metallothionein promoter, murine mammary tumor virus promoter, Rous sarcoma virus promoter, polyhedrin promoter, or other promoters shown effective for expression in eukaryotic cells.

Recombinant viral vectors may also be used to transduce (i.e. infect) the mesenchymal stem cells of the present invention. Viruses are very specialized infectious agents that have evolved, in many cases, to elude host defense mechanisms. Typically, viruses infect and propagate in specific cell types. The targeting specificity of viral vectors utilizes its natural specificity to specifically target predetermined cell types and thereby introduce a recombinant gene into the infected cell.

The present inventors have shown that retroviruses (e.g. lentivirus) may be used to efficiently transduce mesenchymal stem cells with the anti angiogenic factor PEX.

Retroviral constructs of the present invention may contain retroviral LTRs, packaging signals, and any other sequences that facilitate creation of infectious retroviral vectors. Retroviral LTRs and packaging signals allow the polypeptides of the invention to be packaged into infectious particles and delivered to the cell by viral infection. Methods for making recombinant retroviral vectors are well known in the art (see for example, Brenner et al., PNAS 86:5517-5512 (1989); Xiong et al., Developmental Dynamics 212:181-197 (1998) and references therein).

Examples of retroviral sequences useful in the present invention include those derived from adenovirus, lentivirus, Herpes simplex I virus, or adeno-associated virus (AAV). Other viruses known in the art are also useful in the present invention and therefore will be familiar to the ordinarily skilled artisan.

Various methods can be used to introduce the expression vector of the present invention into cells. Such methods are generally described in Sambrook et al., Molecular Cloning: A Laboratory Manual, Cold Springs Harbor Laboratory, New York (1989, 1992), in Ausubel et al., Current Protocols in Molecular Biology, John Wiley and Sons, Baltimore, Md. (1989), Chang et al., Somatic Gene Therapy, CRC Press, Ann Arbor, Mich. (1995), Vega et al., Gene Targeting, CRC Press, Ann Arbor Mich. (1995), Vectors: A Survey of Molecular Cloning Vectors and Their Uses, Butterworths, Boston Mass. (1988) and Gilboa et at. [Biotechniques 4 (6): 504-512, 1986] and include, for example, stable or transient transfection, lipofection, electroporation and infection with recombinant viral vectors. In addition, see U.S. Pat. Nos. 5,464,764 and 5,487,992 for positive-negative selection methods.

Fresh or frozen (e.g., cryo-preserved) preparations may be employed.

Depending on the medical condition, the subject may be administered with additional chemical drugs (e.g., immunomodulatory, chemotherapy etc.) or cells.

Preferably the HSCs and stromal cells share some common HLA antigens.

Examples of immunosuppressive agents include, but are not limited to, methotrexate, cyclophosphamide, cyclosporine, cyclosporin A, chloroquine, hydroxychloroquine, sulfasalazine (sulphasalazopyrine), gold salts, D-penicillamine, leflunomide, azathioprine, anakinra, infliximab (REMICADE), etanercept, TNF.alpha. blockers, a biological agent that targets an inflammatory cytokine, and Non-Steroidal Anti-Inflammatory Drug (NSAIDs). Examples of NSAIDs include, but are not limited to acetyl salicylic acid, choline magnesium salicylate, diflunisal, magnesium salicylate, salsalate, sodium salicylate, diclofenac, etodolac, fenoprofen, flurbiprofen, indomethacin, ketoprofen, ketorolac, meclofenamate, naproxen, nabumetone, phenylbutazone, piroxicam, sulindac, tolmetin, acetaminophen, ibuprofen, Cox-2 inhibitors and tramadol.

In any of the methods described herein, the cells or media can be administered either *per se* or, preferably as a part of a pharmaceutical composition that further comprises a pharmaceutically acceptable carrier.

As used herein a "pharmaceutical composition" refers to a preparation of one or more of the chemical conjugates described herein, with other chemical components such as pharmaceutically suitable carriers and excipients. The purpose of a pharmaceutical composition is to facilitate administration of a compound to a subject.

Hereinafter, the term "pharmaceutically acceptable carrier" refers to a carrier or a diluent that does not cause significant irritation to a subject and does not abrogate the biological activity and properties of the administered compound. Examples, without limitations, of carriers are propylene glycol, saline, emulsions and mixtures of organic solvents with water.

Herein the term "excipient" refers to an inert substance added to a pharmaceutical composition to further facilitate administration of a compound. Examples, without limitation, of excipients include calcium carbonate, calcium phosphate, various sugars and types of starch, cellulose derivatives, gelatin, vegetable oils and polyethylene glycols.

According to a preferred embodiment of the present invention, the pharmaceutical carrier is an aqueous solution of saline.

Techniques for formulation and administration of drugs may be found in "Remington's Pharmaceutical Sciences," Mack Publishing Co., Easton, PA, latest edition.

One may administer the pharmaceutical composition in a systemic manner (as detailed hereinabove). Alternatively, one may administer the pharmaceutical composition locally, for example, via injection of the pharmaceutical composition directly into a tissue region of a patient.

Pharmaceutical compositions of the present invention may be manufactured by processes well known in the art, e.g., by means of conventional mixing, dissolving, granulating, dragee-making, levigating, emulsifying, encapsulating, entrapping or lyophilizing processes.

Pharmaceutical compositions for use in accordance with the present invention thus may be formulated in conventional manner using one or more physiologically acceptable carriers comprising excipients and auxiliaries, which facilitate processing of the active ingredients into preparations which, can be used pharmaceutically. Proper formulation is dependent upon the route of administration chosen.

For injection, the active ingredients of the pharmaceutical composition may be formulated in aqueous solutions, preferably in physiologically compatible buffers such as Hank's solution, Ringer's solution, or physiological salt buffer. For transmucosal administration, penetrants appropriate to the barrier to be permeated are used in the formulation. Such penetrants are generally known in the art.

For any preparation used in the methods of the invention, the therapeutically effective amount or dose can be estimated initially from *in vitro* and cell culture assays. Preferably, a dose is formulated in an animal model to achieve a desired concentration or titer. Such information can be used to more accurately determine useful doses in humans.

Toxicity and therapeutic efficacy of the active ingredients described herein can be determined by standard pharmaceutical procedures *in vitro,* in cell cultures or experimental animals.

The data obtained from these *in vitro* and cell culture assays and animal studies can be used in formulating a range of dosage for use in human. The dosage may vary depending upon the dosage form employed and the route of administration utilized. The exact formulation, route of administration and dosage can be chosen by the individual physician in view of the patient's condition, (see e.g., Fingl, et al., 1975, in "The Pharmacological Basis of Therapeutics", Ch. 1 p.1). For example, Parkinson's patient can be monitored symptomatically for improved motor functions indicating positive response to treatment.

For injection, the active ingredients of the pharmaceutical composition may be formulated in aqueous solutions, preferably in physiologically compatible buffers such as Hank's solution, Ringer's solution, or physiological salt buffer.

Dosage amount and interval may be adjusted individually to levels of the active ingredient which are sufficient to effectively regulate the neurotransmitter synthesis by the implanted cells. Dosages necessary to achieve the desired effect will depend on individual characteristics and route of administration. Detection assays can be used to determine plasma concentrations.

Depending on the severity and responsiveness of the condition to be treated, dosing can be of a single or a plurality of administrations, with course of treatment lasting from several days to several weeks or diminution of the disease state is achieved.

The amount of a composition to be administered will, of course, be dependent on the individual being treated, the severity of the affliction, the manner of administration, the judgment of the prescribing physician, etc. The dosage and timing of administration will be responsive to a careful and continuous monitoring of the individual changing condition. For example, a treated Parkinson's patient will be administered with an amount of cells which is sufficient to alleviate the symptoms of the diseas

In order to ensure vascularization of the encapsulated cells, pre-vascularized solid supports may be used to improve to nutrition of the encapsulated cells (DE Vos Trend. Mol. Med. 2002 363-366.

Toxicity and therapeutic efficacy of the active ingredients described herein can be determined by standard pharmaceutical procedures in experimental animals.

For example, 6-OHDA-lesioned mice may be used as animal models of Parkinson's. In addition, a sunflower test may be used to test improvement in delicate motor function by challenging the animals to open sunflowers seeds during a particular time period.

Transgenic mice may be used as a model for Huntingdon's disease which comprise increased numbers of CAG repeats have intranuclear inclusions of huntingtin and ubiquitin in neurons of the striatum and cerebral cortex but not in the brain stem, thalamus, or spinal cord, matching closely the sites of neuronal cell loss in the disease.

Transgenic mice may be used as a model for ALS disease which comprise SOD-1 mutations.

The septohippocampal pathway, transected unilaterally by cutting the fimbria, mimics the cholinergic deficit of the septohippocampal pathway loss in Alzheimers disease. Accordingly animal models comprising this lesion may be used to test the cells of the present invention for treating Alzheimers.

In general, schizophrenia animal models can be divided in three categories, i.e. models that investigate behaviours in animals that are disturbed in schizophrenic patients (e.g. prepulse inhibition of the acoustic startle response and latent inhibition), pharmacological models, and experimentally induced brain pathology e.g. brain lesion models. Methods of generating such models and use of same are described in Bachevalier, J. (1994) Medial temporal lobe structures and autism, a review of clinical and experimental findings. Neuropsychologia 32, 627-648; R.Joober et al. Genetic of schizophrenia: from animal models to clinical studies. J.Psychiatry Neurosci. 2003; 27 (5): 336-47; Lipska, B.K., Jaskiw, G.E., Weinberger, D.R., 1993, Postpuberal emergence of hyperresponsiveness to stress and to amphetamine after neonatal hippocampal damage, a potential animal model for schizophrenia. Neuropsychopharmacol. 122, 35-43; Weinberger, R.R. (1987) Implications of normal brain development for the pathogenesis of schizophrenia. Arch. Gen. Psychiatry 44: 660-669; Wolterink G., Daenen, E.W.P.M., Dubbeldam, S., Gerrits, M.A.F.M., Van Rijn, R., Kruse, C.G., Van der Heijden, J., Van Ree, J.M. (2001) Early amygdala damage in the rat as model for neurodevelopmental psychopathological. Eur. Neuropsychopharmacol. 11, 51-59; and Daenen E.W.P.M., Wolterink G., Gerrits M.A.F.M., Van Ree J.M. (2002) Amygdala or ventral hippocampal lesions at two early stages of life differentially affect open filed behaviour later in life: an animal model of neurodevelopmental psychopathological disorders. Behavioral Brain Research 131: 67-78.

The data obtained from these animal studies can be used in formulating a range of dosage for use in human. The dosage may vary depending upon the dosage form employed and the route of administration utilized. The exact formulation, route of administration and dosage can be chosen by the individual physician in view of the patient's condition. (See e.g., Fingl, et al., 1975, in "The Pharmacological Basis of Therapeutics", Ch. 1 p.1).

Following transplantation, the cells of the present invention preferably survive in the diseased area for a period of time (e.g. at least 6 months), such that a therapeutic effect is observed.

Compositions including the preparation of the present invention formulated in a compatible pharmaceutical carrier may also be prepared, placed in an appropriate container, and labeled for treatment of an indicated condition.

Compositions of the present invention may, if desired, be presented in a pack or dispenser device, such as an FDA approved kit, which may contain one or more unit dosage forms containing the active ingredient. The pack may, for example, comprise metal or plastic foil, such as a blister pack. The pack or dispenser device may be accompanied by instructions for administration. The pack or dispenser may also be accommodated by a notice associated with the container in a form prescribed by a governmental agency regulating the manufacture, use or sale of pharmaceuticals, which notice is reflective of approval by the agency of the form of the compositions or human or veterinary administration. Such notice, for example, may be of labeling approved by the U.S. Food and Drug Administration for prescription drugs or of an approved product insert.

Thus, compositions of some embodiments of the present invention are designed to protect MSCs from the immune system and act as a mini-bioreactor, which will allow cells to secrete endogenous or exogenous factors at or near the site of interest.

As used herein the term "about" refers to ± 10 %.

The terms "comprises", "comprising", "includes", "including", "having" and their conjugates mean "including but not limited to".

The term "consisting of means "including and limited to".

The term "consisting essentially of" means that the composition, method or structure may include additional ingredients, steps and/or parts, but only if the additional ingredients, steps and/or parts do not materially alter the basic and novel characteristics of the claimed composition, method or structure.

As used herein, the singular form "a", "an" and "the" include plural references unless the context clearly dictates otherwise. For example, the term "a compound" or "at least one compound" may include a plurality of compounds, including mixtures thereof.

Throughout this application, various embodiments of this invention may be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

Whenever a numerical range is indicated herein, it is meant to include any cited numeral (fractional or integral) within the indicated range. The phrases "ranging/ranges between" a first indicate number and a second indicate number and "ranging/ranges from" a first indicate number "to" a second indicate number are used herein interchangeably and are meant to include the first and second indicated numbers and all the fractional and integral numerals therebetween.

As used herein the term "method" refers to manners, means, techniques and procedures for accomplishing a given task including, but not limited to, those manners, means, techniques and procedures either known to, or readily developed from known manners, means, techniques and procedures by practitioners of the chemical, pharmacological, biological, biochemical and medical arts.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination or as suitable in any other described embodiment of the invention. Certain features described in the context of various embodiments are not to be considered essential features of those embodiments, unless the embodiment is inoperative without those elements.

Various embodiments and aspects of the present invention as delineated hereinabove and as claimed in the claims section below find experimental support in the following examples.

### EXAMPLES

Reference is now made to the following examples, which together with the above descriptions illustrate some embodiments of the invention in a non limiting fashion.

Generally, the nomenclature used herein and the laboratory procedures utilized in the present invention include molecular, biochemical, microbiological and recombinant DNA techniques. Such techniques are thoroughly explained in the literature. See, for example, "Molecular Cloning: A laboratory Manual" Sambrook et al., (1989); "Current Protocols in Molecular Biology" Volumes I-III Ausubel, R. M., ed. (1994); Ausubel et al., "Current Protocols in Molecular Biology", John Wiley and Sons, Baltimore, Maryland (1989); Perbal, "A Practical Guide to Molecular Cloning", John Wiley & Sons, New York (1988); Watson et al., "Recombinant DNA", Scientific American Books, New York; Birren et al. (eds) "Genome Analysis: A Laboratory Manual Series", Vols. 1-4, Cold Spring Harbor Laboratory Press, New York (1998); methodologies as set forth in U.S. Pat. Nos. 4,666,828; 4,683,202; 4,801,531; 5,192,659 and 5,272,057; "Cell Biology: A Laboratory Handbook", Volumes I-III Cellis, J. E., ed. (1994); "Current Protocols in Immunology" Volumes I-III Coligan J. E., ed. (1994); Stites et al. (eds), "Basic and Clinical Immunology" (8th Edition), Appleton & Lange, Norwalk, CT (1994); Mishell and Shiigi (eds), "Selected Methods in Cellular Immunology", W. H. Freeman and Co., New York (1980); available immunoassays are extensively described in the patent and scientific literature, see, for example, U.S. Pat. Nos. 3,791,932; 3,839,153; 3,850,752; 3,850,578; 3,853,987; 3,867,517; 3,879,262; 3,901,654; 3,935,074; 3,984,533; 3,996,345; 4,034,074; 4,098,876; 4,879,219; 5,011,771 and 5,281,521; "Oligonucleotide Synthesis" Gait, M. J., ed. (1984); "Nucleic Acid Hybridization" Hames, B. D., and Higgins S. J., eds. (1985); "Transcription and Translation" Hames, B. D., and Higgins S. J., Eds. (1984); "Animal Cell Culture" Freshney, R. I., ed. (1986); "Immobilized Cells and Enzymes" IRL Press, (1986); "A Practical Guide to Molecular Cloning" Perbal, B., (1984) and "Methods in Enzymology" Vol. 1-317, Academic Press; "PCR Protocols: A Guide To Methods And Applications", Academic Press, San Diego, CA (1990); Marshak et al., "Strategies for Protein Purification and Characterization - A Laboratory Course Manual" CSHL Press (1996). Other general references are provided throughout this document. The procedures therein are believed to be well known in the art and are provided for the convenience of the reader.

### GENERAL MATERIALS AND EXPERIMENTAL METHODS

***Isolation of hMSCs from bone marrow -*** Fresh human bone marrow (Cambrex, USA) was kindly given by Dr. Erella Livne from the Rappaport Department of Medicine, the Technion, Israel Institute of Technology (This study was approved by the Helsinki Committee Board of the Technion Faculty of Medicine, Haifa, Israel). The bone marrow was washed twice with phosphate buffered saline (PBS) at room temperature upon arrival and bone marrow-derived MSCs were isolated based on their adhesion to polysterene, cultured (37 °C, 5 % CO₂, 1 week) in standard medium [DMEM low glucose (1000 mg/ml) (Biological Industries, Israel), 10 % fetal calf serum (FCS), 2 mM L-glutamine and Pen-Strep (100 U/ml, 100 µg/ml) (Biological Industries, Israel)]. At 70-80 % confluence the cells were trypsinized and re-plated [Passage 1 (P₁)] for an additional week.

***Cell culture -*** hMSCs were cultured in Dulbecco's modified Eagle's Medium (DMEM) low glucose (1000 mg/ml; Biological Industries, Israel), supplemented with 2 mM L-glutamine, Pen-Strep (100 U/ml, 100 µg/ml; Biological Industries, Israel), fungizon (Gibco, USA) and 10 % fetal calf serum (FCS; Gibco, USA). The hMSCs were usually plated onto tissue culture dishes at a density of 5,000 to 6,000 cells per cm² growth area. The medium was changed every 3-4 days. When an 80 % confluence was reached, the cells were subcultured by trypsinization (0.25 % trypsin-EDTA) or deep freezed for future use (freezing medium: 90 % FCS and 10 % dimethyl sulfoxide in liquid N₂). Most of the experiments were conducted till cell passage 5-6. The hMSCs were cultured as mono-layers in a humidified atmosphere of 95 % air and 5 % CO₂.

NIH-3T3 (ATCC, CRL-1658 USA), Hek-293 (ATCC CRL-1573, USA) and U-87 human blastoglioma (ATCC, HTB-14, USA) were cultured in Dulbecco's modified Eagle's Medium (DMEM) high glucose (4500 mg/ml; Biological Industries, Israel), supplemented with 2 mM L-glutamine, Pen-Strep (100 U/ml, 100 µg/ml; Biological Industries, Israel), fungizon (Gibco, USA) and 10 % fetal calf serum (FCS; Gibco, USA).

***hMSCs morphology analysis -*** To study the morphology of the hMSCs, 15,000 cells were seeded onto 6-well culture plates over a cover glass in complete growth medium and were allowed to attach overnight. The following day, the cells were washed with PBS and fixed with 4 % formaldehyde. The cells were washed again and were subject to either Dio/DAPI fluorescent staining or morphological staining with Giemsa. For fluorescent staining, the Dio^{®} (Dako, diluted 1:1000) fluorescent dye, which stains the cell's cytoplasm, and the Dapi^{®} (Dako, diluted at 0.5 µg/ml) fluorescent dye, which stains the cell's nuclei, were applied. Stained cells were washed and the coverslip was mounted over a slide with fluoromount G™ (Soutern Biotech, Birminghm, AL USA), a quenching reducer. For the morphological staining, the hMSCs were stained for 5 minutes with Wright stains (Sigma Aldrich), washed twice with PBS and stained for additional 5 minutes with Giemsa stains (Sigma Aldrich). Cells were visualized using inverted fluorescent microscope (TE2000-S, Nikon). Images were analyzed using Lucia software (Laboratory Imaging, ZA Drahav, Prague, CZ).

***Cell encapsulation in alginate - poly L(lysine) (PLL) microcapsules -*** Alginate (LVG (low viscosity high guluronic acid content) or MVG (moderate viscosity high guluronic acid content, Pronova, Norway) was dissolved in saline at concentrations of 2 % or 3 % [weight per volume (w/v), such that the stock solution was 2-3% while the working solution was 1.2%] by stirring for 24 hours at room temperature. The solution was then kept at 4 °C. Cells were trypsinized, counted, and suspended in serum free growth media (e.g., the the same as descried in cell culture section growth medium available from Biological Industries, Israel). hMSCs were suspended in 1.2 % (w/v) sodium alginate solution. Encapsulated cell concentration was optimized using different cell ratios (0.75-1.5 x 10⁶ cells per 1 ml of alginate). The cell suspension was sprayed in a steady flow rate of 5 ml/minute using a syringe pump (Harvard Apparatus, Holliston, MA, USA, Catalogue number 70-2208) through a 20 Gauge needle located inside an air jet-head droplet forming apparatus into a HEPES buffered calcium chloride [13 mM HEPES, 1.5 % (w/v) CaCl₂, pH 7.4] solution and allowed to gel for 20 minutes. Alginate microcapsules were then covered with 0.06 % (w/v) PLL of 28.2 kDa (Sigma Aldrich, Catalogue No. P7890) in saline for 10 minutes with gentle agitation. The microcapsules were washed three times in HEPES and cultured using appropriate medium at 37 °C, 5 % CO₂ incubator. The cell encapsulation process is schematically depicted in Figure 1.

***Calcium chelation -*** Liquefaction of the microcapsules core was performed using an additional step of suspension in 0.05 mM sodium citrate (Sigma Aldrich) solution for 10 minutes.

NIH-3T3 cells were encapsulated in alginate-PLL as previously described above with respect to the hMSCS to a final ratio of 1 x 10⁶ cells per 1 ml of alginate.

***Microcapsules cultures -*** Microcapsules were placed in 10 cm tissue culture plates with 10 ml of appropriate medium. In each plate, 2 x 10⁶ encapsulated cells were cultured. Microcapsules were incubated at 37 °C with 5 % CO₂ and the condition media was replaced every 3 to 4 days.

***FACS analyses for hMSCs surface markers analysis -*** Aliquots (2 x 10⁵ cells) of hMSCs (Ps.3-7) were used separately for the analysis of cell surface markers. The cells were washed with 1 x PBS, fixated with 0.5 % formaldehyde in PBS for 10 minutes at 37 °C. The cells were then washed in PBS and resuspended in wash buffer consisting of 0.5 % bovine serum albumin (BSA) in PBS. The cells were incubated with mouse anti human CD105 (Becton Dickson 555690), CD31 (555444 Becton Dickson), CD90 (555593 Becton Dickson), CD44 (555470 Becton Dickson), CD29 (555442 Becton Dickson), CD133 (Milteny Biotec, 130-050-801), CD144 (555661 Becton Dickson), and CD34 (555820, Becton Dickson) monoclonal antibodies for 30 minutes at room temperature (RT). The cells were then washed twice with the wash buffer and stained with FITC conjugated goat anti mouse antibody (Becton Dickson; 555988) and incubated for another 30 minutes. The cells were washed with the wash buffer, resuspended in 0.5 ml of the wash buffer and analyzed for the expression of the abovementioned human antigens by using FACScan and CellQuest software for data collection and analysis (Becton Dickson).

***Encapsulated cell viability and proliferation assays -*** The viability of the encapsulated hMSCs and NIH-3T3 cells was assessed using the fluorimetric qualitative fluorescein diacetate assay (FDA) and the fluorimetric quantitative Alamar-Blue^{®} assay (Serotec, UK). For the FDA test, capsules were incubated for 10 minutes in a 0.67 µg/ml FDA (Sigma Aldrich), washed twice in HEPES and then were observed under a fluorescent microscope (Nikon TE2000-S, Nikron Corporation, Tokyo, Japan). The Alamar-Blue^{®} assay was performed according to the manufacturer's instructions, with slight modifications for encapsulated cells. Briefly, 100,000 encapsulated cells (cell density at the encapsulation time point) were incubated in a 24 well culture plates with the Alamar-Blue^{®} solution to a final concentration of 10 % in culture medium containing microcapsules for four hours. After incubation 100 µl of the condition media was collected into a 96-well culture plate. The fluorescence intensity was quantified by fluorimeter (excitation 535 nm, emission 590 nm).

The proliferation of the encapsulated hMSCs and NIH-3T3 cells was assessed using the H³ Thymidine assay (Amersham Pharmacia Biotech, UK). Briefly, 100,000 encapsulated cells (cell density at the encapsulation time point) were incubated in a 24 well culture plates with the H³ Thymidine solution to a final concentration of 10 µCi/ml in culture medium containing microcapsules for 24 hours. After incubation the microcapsules were washed twice with PBS and resuspended in 1 ml of a dissolving solution (NaOH 0.4 M in PBS-EDTA) for 10 minutes with slight agitation. The dissolved microcapsules were spun down and 100 µl of the upper media was collected into 4 ml scintillation liquid (Perkin Elmer). The sample was mixed and UV intensity was quantified using a β counter.

As a control, the viability/proliferation of 100,000 non-encapsulated cells was measured 12 hours after initial seeding. The viability/proliferation of encapsulated cells is presented as the percentage of viable cells in microcapsules at the different time points relative to the viability/proliferation of control cells (non-encapsulated cells), which is indicated as 100 % viability/proliferation.

***In vitro immunogenesity studies -*** Cells isolated from mouse spleens are cultured with microcapsules containing stem cells or control cells. The proliferation of spleen cells is evaluated using an AlamarBlue assay. Cytokine and total IgG and IgM is monitored with ELISA and ELISPOT assay kits in order to evaluate the immune cell stimulation.

***In vitro encapsulated cells monocyte activation -*** Monocyte activation is generally the main reason for the creation of fibrotic tissue around the microcapsules and is involved in the initiation of an inflammatory response. Monocyte activation is studied using several methods: Up regulation of co-stimulatory receptors such as CD80 and CD86, inflammatory cytokine secretion such as: TNF-α, and IL-1β, and by reactive oxygen intermediates such as NO (nitric oxide). The monocyte source is mouse abdominal cavities with no tioglycolate activation. Up regulation of co-stimulatory receptors can be measured using FACS analysis. In all of the experiments, empty microcapsules is used as an additional control group.

### Differentiation assays of encapsulated hMSCs

***Osteogensis assay -*** To induce osteogenic differentiation *in vitro*, human MSCs which were encapsulated in alginate PLL microcapsules were cultured in the presence of an osteogenic induction medium which comprises the maintenance medium (DMEM containing 10 % FCS) supplemented with 0.05 mM ascorbic acid-2-phosphate, 10 mM β-glycerophosphate, and 0.1 µM dexamethasone (sigma). Control encapsulated hMSCs were cultured in the maintenance medium (DMEM containing 10 % FCS). At 1-3 weeks after addition of the supplement, a portion of the microcapsules was stained with Von Kossa dyes for detection of deposits of calcium according to the protocol. The remaining microcapsules were homogenized in 1.5 % Na-citrate solution for 5-10 minutes, and the retrieved hMSCs were replated on round cover slips and incubated for additional 24 hours. The retrieved hMSCs were fixated using 4 % paraformaldehyde (PFA) for 10 minutes, washed and stained for alkaline phosphatase (ALP) activity according to the protocol.

***Adipogenic assay*** - To induce adipogenic differentiation *in vitro,* human MSCs which were encapsulated in alginate PLL microcapsules were cultured in the presence of either a high glucose DMEM containing 10 % FCS, 1 µM dexamethasone, 10 µM insulin, 0.5 mM 3-isobutyl-1-methylxanthine, and 100 µM indomethacin (induction medium) or high glucose DMEM containing 10 % FCS and 10 µM insulin (Maintenance medium). The "adipo-differentiated" microcapsules were cultured in induction medium for 3 days followed by 3 days in maintenance medium. The microcapsules underwent 3 cycles before they were assayed for adipogenic differentiation. The control microcapsules were cultured in maintenance medium for the entire period of differentiation. At the end of the assay, the microcapsules were either stained directly using Oil Red O staining according to the protocol (Sigma Aldrich) or homogenized as descried earlier. The retrieved hMSCs were fixated and stained using Oil Red O staining.

***Chondrogensis assay*** - To induce chondrogenic differentiation *in vitro,* human MSCs which were encapsulated in alginate PLL microcapsules were treated for 10 minutes with 1.5 % Na-citrate to allow the creation of cell aggregates. The "chondro-differentiation" microcapsules were cultured in DMEM containing 10 % FCS, 50 mM ascorbic acid-2-phosphate, 0.1 µM dexamethasone, 1 mM sodium pyruvate and 10 ng/ml TGF-β. The control microcapsules were cultured in DMEM containing 10 % FCS. After 3 weeks the microcapsules were stained directly using Alcian Blue staining according to the protocol (Sigma Aldrich) or homogenized as descried earlier. The retrieved hMSCs were fixated and stained using Alcian Blue staining.

***Constriction of genetically modified hMSCs expressing the anti angiogenic factor PEX*** - Genetically modified hMSCs were generated by lentivirus transduction. The lentivirus vectors CSCW2-IRES-mCherry and CSCW2-PEX-IRES-mCherry were kindly given by Dr. Rona Carroll from the Brigham and Women's Hospital, Boston, MA. The vectors were amplified in xl-1 blue cells and purified using Purelink^{™}, HiPure plasmid maxiprep (Invitrogen). The lentivirus was packaged in 293FT cell using the ViraPower^{™}, Lentiviral Expression System following the manufacture's protocol (invitrogen). HMSC were transduced with viral supernatant with titer of 1:5 and 6 µg/ml polybrene (Sigma).

The expression of the m-Cherry was verified using florescence microscope (excitation 587 nm, emission 610 nm). The expression of PEX was verified using RT-PCR and Western blotting as further described below.

### Biological activity of encapsulated hMSCs expressing exogenous PEX

***Cell Proliferation*** - Proliferation assays in the presence of microcapsules entrapping PEX secreting hMSCs were performed on Human Umbilical Vein Endothelial Cells (HUVEC) and U87 cells using Thymidine incorporation assay. Cells (20,000/well) were seeded on 24 well plates, and maintained over night with the suitable medium, supplemented with 5 % FCS, and antibiotic. Transwell membrane (Corning, CAT NO 3470) inserts were then placed within the wells, and microcapsules were placed on the Transwell membrane. After 48 hours, H³ thymidine (1 µCi/ml) was added to each well, and cells were incubated for another 12 hours. The cells were then washed three times with PBS and lysed with 300 µl of 0.2 N NaOH for 20 minutes. Samples were then placed in 4 ml scintillation liquid and analyzed with β-counter for radioactivity [count per minute (CPM)].

### In vivo studies

***Biocompatibility analysis of the Alginate PLL Microcapsules -** In vivo* studies were conducted in order to evaluate the biocompatibility of the developed alginate PLL microcapsules entrapping MSCs. Six-week old C57 black female mice were transplanted subcutaneously with the microcapsules. After transplantation, blood is collected and tested for total IgG using ELISA kit. Mice were sacrificed at four time points following transplantation: one week, two weeks, four weeks and eight weeks. Each treatment group consisted of 24 mice, 6 mice were sacrificed at each time point.

Treatment groups were as follows:
- Shem operation - PBS only,
- Alginate PLL alginate microcapsules - no cells
- Alginate PLL alginate microcapsules entrapping HEK-293 cell line
- Alginate PLL alginate microcapsules entrapping human MSCs (hMSCs)
- Alginate PLL alginate microcapsules entrapping rat MSCs (rMSCs)

***Evaluation of retrieved microcapsules from transplanted animals*** - At each scarification time point, pictures of the microcapsules within the mouse were taken prior to retrieval of the microcapsules. Half of the retrieved microcapsules were placed in condition medium, stained with FDA and observed under light and florescence microscope to evaluate their morphology, viability and tissue over growth. The other half of the retrieved microcapsules were used for paraffin embedding. For the evaluation of fibrotic tissue, the retrieved microcapsules were tested by histological and immunohistochemical assays:
(1) Hematoxylin-Eosin (H&E): The H&E staining method enables the identification of cell type, morphology, and presence of cells active in the inflammatory process such as macrophages, NK and dendritic cells.
(2) Immunohistochemistry - Because TNF-α, and IL-1β, are considered to be major inflammatory cytokines, TNF-α, and IL-1β specific antibodies are used with biotinylated secondary antibodies. The colorimetric reaction is performed using strepavidin conjugated AP or HRP.

***Evaluation of receipient tissues after transplantation with the microcapsules*** - The following fluid/tissues/organs were taken out of sacrificed mice: Blood, liver, spleen, inguinal lymph nodes and skin from the transplantation site.

The removed tissues are detected for the presence of cytokine levels by RT-PCR analysis and histological staining.

***Immunohistochemistry assays*** - For immunohistochemistry, tumor and skin specimens were embedded in OCT, frozen on dry ice, and stored at -80 °C. Frozen sections (5 µM) were cut using a cryostat (Lucia). Sections of each specimen were stained using H&E. Immunohistochemistry was carried out using the Vectastain Elite ABC kit (Vector Laboratories). Primary antibodies included CD31 (ab28364), Ki-67 nuclear antigen (ab16667), F4-80 (ab6640) and CD69 (ab25190, abcam, Cambridge, MA, USA) Detection was carried out using a DAB chromogen, which resulted in a positive brown staining. Sections were counterstained with hematoxylin, dehydrated in ethanol, and mounted with glass cover slips. The staining was quantified by counting the number of positively stained cells of all nuclei in 20 randomly chosen fields. Microcapsules were fixated in 10 % formalin, washed twice and dehydrated using elevated concentrations of ethanol. Then, the microcapsules were embedded in paraffin molds and cut (5 µM) using a microtome. Sections then undergo deparaffinization and stained using H&E staining.

***RT-PCR analysis of cells or tissues' RNA*** - To study the expresison level of PEX from PEX-transduced hMSCs the RNA was extracted from the cells using the Tri-reagent (Sigma Aldrich) according to manufacturer's protocol.

To study the immunogenicity of the encapsulated MSC a real time PCR analysis was performed. C57 black mice inguinal lymph nodes from both sides were harvested and processed using homogenizer. RNA was extracted using the Tri-reagent.

RNA was suspended in diethylpyrocarbonate (DEPC) treated water, and quantified at 260 nm. 1 µg from the RNA solution was treated with DNase (RNase free) for 30 minutes at 35 °C. The DNA free RNA was used in the 1^{st} strand AB-Gene kit for synthesis of first strand cDNA, using random primers from the kit.

The PCR reaction was prepared by combining 2-4 µl from the first cDNA template, 1 µl from each of the primers (Table 1), 4 µl of x5 ready mix and double distilled water (DDW) for total volume of 20 µl. The PCR was set for 35 cycles of 95 °C for 30 seconds, 55 °C for 30 seconds, and 72 °C for 60 seconds. PCR products were analyzed by 1 % agarose gel electrophoresis with EtBr staining.

Table 1, hereinbelow, provides a list of primers (and their SEQ ID NO:) used for PCR amplification. The RT-PCR results are shown as relative cycle number of IL-1β or TNF-α to GAPDH and to control.

**Table 1**

| ***PCR primers*** | | |
|---|---|---|
| ***GENE (GenBank Accession No. of mRNA)*** | ***Primer name (SEQ ID NO:)*** | ***Sequence (5'→3')*** |
| PEX (YP 001228336.1) | PEX reverse (SEQ ID NO:1) | 5' - ACTTTGGTTCTCCAGCTTC-3' |
| | PEX forward (SEQ ID NO:2) | 5' - ATTGATGCGGTATACCAGG-3' |
| Interleukin-1-beta (IL-1 β (NM 204524.1) | IL-1-β forward (SEQ ID NO:3) | 5' - CATGGAATCTGTGTCTTCCTAAAGT |
| | IL-1-β reverse (SEQ ID NO:4) | 5'-GTTCTAGAGAGTGCTGCCTAATGTC |
| Tumor necrosis factor alpha (TNF-α) (NM 000594.2) | TNF-α forward (SEQ ID NO:5) | 5' - GATTTGCTATCTCATACCAGGAGAA |
| | TNF-α reverse (SEQ ID NO:6) | 5' - GACAATAAAGGGGTCAGAGTAAAGG |
| GAPDH (NM 017008) | GAPDH forward (SEQ ID NO:7) | 5' - ACCCAGAAGACTGTGGATGG |
| | GAPDH reverse (SEQ ID NO:8) | 5' - CTTGCTCAGTGTCCTTGCTG |

### EXAMPLE 1

### DEVELOPING A STEM CELL ENCAPSULATION SYSTEMACHIEVING LONG-TERMSURVIVAL OF THE CELLS

The present inventors have devised a stem cell encapsulation system which includes adult stem cells [e.g., neural stem cells (NSC) and mesenchymal stem cells (MSC)] and embryonic stem cells (ESC). Encapsulation was based on alginate and PLL, two biocompatible polymers and the viability and proliferation, encapsulated cell morphology, cell marker phenotype, renewal capability (telomerase activity), and multilinage differentiation potential were followed over time, as follows.

Encapsulated cell morphology is conducted using light microscopy and fluorescence staining. The encapsulated cell marker phenotype is observed by liquefaction of the alginate beads or alginate-PLL microcapsules. Retrieved cells are replated on slides, fixated with 4 % paraformaldehyde (PFA) and incubated with a specific cell marker antibody. Secondary staining is performed using fluorescence or colorimeter antibodies. Renewal capability is examined using a telomere repeat amplification protocol (TRAP). Multilinage differentiation potential is examined by retrieving the encapsulated cells and growing them with the appropriate differentiation medium. Marker characterization of the stem cells is performed using flow cytometry analysis. The cells will be fluorescently labeled using specific cell surface marker antibodies. Cells are analyzed with a FACS cytometer and WinMDI software. The number of cells encapsulated in the microcapsules is an important factor that needs to be taken in account when considering long-term cell encapsulation. Higher cell densities may lead to cell necrosis and lack of protein synthesis. On the other hand, lower cell densities may not be sufficient in order to achieve a substantial drug production. When choosing the right cell number for encapsulation it is important to examine the growth behavior of the cell source before and after encapsulation. The viability, proliferation, and growth morphology of the various stem cells is studied in correlation to the number of cells encapsulated. Encapsulated cell viability and proliferation is measured using different assays such as AlamarBlue, FDA/PI viability assay, and modified H³ -Thymidine assay.

### EXAMPLE 2

### ENCAPSULATED STEM CELLS ARE VIABLE AND PROLIFERATIVE

HMSC were subdivided from whole bone marrow specimens by cell adhesion. The cells were cultured and grown till passage 7-8. The stem cells of the invention were encapsulated in alginate-PLL microcapsules as schematically shown in Figure 1.

### Experimental Results

***Encapsulation of human mesenchymal stem cell in alginate-PLL Microcapsules -*** Human MSCs were encapsulated in alginate-PLL microcapsules (Figures 2a-c). The microcapsules had an average diameter of 0.5 mm ± 0.08 mm (Figures 4a-d). The alginate poly(L-lysine) microcapsules enable access of nutrients from the medium to the encapsulated cells as well as secretion of cells-derived material (e.g., proteins, therapeutics) from the microcapsules to the medium. On the other hand, due to the alginate-PLL encapsulation, the cells do not induce an immune response in the host organism. The encapsulated cells maintained the typical morphology of hMSCs (Figures 3a-d and Figure 4a-d).

***Encapsulated stem cells are viable and proliferative -*** As shown in Figures 5a-b, the Alamar blue assay demonstrated that human MSCs are viable in the alginate-PLL microcapsules for at least 70 days post encapsulation, and exhibit viability levels which are higher or similar to that of control, non-encapsulated cells, and are significantly higher than those of encapsulated NIH-3T3 cells (Figure 5a), which are typically used for cell encapsulation.

In addition, as shown in Figure 5b and 6a-d, inverted and florescence micrographs taken on day 28 and 70 post encapsulation had reveled different morphology pattern: both cell types were viable but while the NIH-3T3 cells expanded aggressively inside the microcapsules the hMSCs had grown evenly without creating cell clusters.

***The effect of calcium chelation on encapsulated MSCs -*** The process of calcium chelation renders the capsule empty, thus mimicking a balloon structure. Thus, the encapsulated cells can be in an empty space like the 3-dimensions needed for embryonic stem cells. To test the effect of calcium chelation on the viability and proliferative capacity of the encapsulated human MSCs, alginate-PLL microcapsules were prepared in the presence or absence of the calcium chelator, and the viability and proliferation of cells were measured using Alamar blue assay and H³-Thymidine incorporation assay, respectively. As is shown in Figures 7a-b, no significant difference was observed in the presence of calcium chelation.

### EXAMPLE 3

### ENCAPSULATED STEM CELLS MAINTAIN STEMNESS AND PL URIPOTENT CAPACITY

***Characterization and stem ness of the encapsulated hMSC -*** The encapsulated hMSCs were characterized for their ability to sustain their stem ness properties post encapsulation. Specific surface markers analysis was performed in a similar way to the non encapsulated hMSCs. The cells were retrieved from the microcapsules after 1 and 2 months post encapsulation and specific cell marker analysis was performed using flow cytometry. In a similar way to the non encapsulated hMSCs, the encapsulated hMSCs were positive for CD105, CD90, CD44 and CD29 and negative for CD34, CD133, CD31 and CD 144 with equal and even slightly higher expression levels (Figure 8 and Figures 9a-h).

***Encapsulated stem cells are pluripotent*** - The encapsulated hMSCs were also evaluated for their ability to differentiate into one of the 3 mesoderm lineages: osteoblasts, adipocytes and chondrocytes. The encapsulated hMSCs were grown with the appropriate differentiation media and after 1 and 2 weeks of induction the cells were retrieved and specific staining was performed.

***Encapsulated human MSCs are capable of differentiation to osteoblasts while in capsules*** - Encapsulated human MSCs were cultured in the osteogenic induction medium for 1-3 weeks and then were subjected to in capsule Van-Kossa staining which determines presence of free Calcium (Ca+). As shown in Figures 10a-c (enlarged images of capsules), while the control, undifferentiated hMSCs in capsules, exhibit negligible levels of free calcium (Figure 10a), the encapsulated hMSCs which were cultured for one (Figure 10b) or two (Figure 10c) weeks in the osteogenic induction medium exhibit significant levels of free calcium indicating their in-capsule differentiation into osteoblasts. After retrieving from the capsules, the cells were stained using specific staining reagent (Van kossa which reacts with free calcium ions and the substrate of Alkaline phosphatase which upon cleavage creates colorimetric reaction) and photographed. As shown in Figures 11a-d, following one or two weeks in the osteoblast differentiation medium the encapsulated human MSCs were positive for osteoblast staining, and express typical osteogenic markers such as expression of alkaline phosphatase staining (Figures 11a-b) and secretion of calcium (van kossa staining, Figures 11c-d).

***Encapsulated human MSCs are capable of differentiation to adipocyte while in capsules*** - As is further shown in Figures 12a-b, encapsulated hMSCs which were incubated for 3 weeks in the adipocyte differentiation medium and then retrieved from capsules, exhibit adipocyte markers as indicated by the Oil red O staining.

***Encapsulated As is further shown capable of differentiation to chondrocytes while in capsules*** - As is further shown in Figures 12c-f, encapsulated hMSCs which were incubated for 3 weeks in the chondrocyte differentiation medium and then retrieved from capsules, exhibit chondrogenic characteristics as indicated by the Alcian Blue staining.

### EXAMPLE 4

### TRANSFORMED ENCAPSULATED STEM CELLS MAINTAIN EXOGENOUS EXPRESSION

***Encapsulated human MSCs which are genetically modified to express EGF (Enhanced green fluorescent protein) maintain expression from the exogenous polynucleotide in capsules -*** As shown in Figures 13a-i, even after two months in capsules the transformed cells express the exogenous pEGF polynucleotide

### EXAMPLE 5

### ENCAPSULATED STEM CELLS ARE BIOCOMPATIBEL AND NON-IMMUNOGENIC

The immunogenicity of encapsulated cells is one of the most significant parameters affecting the success of the system *in vivo*. The immunogenicity of the encapsulated adults stem cells (e.g., NSCs and MSCs) and ESCs and of encapsulated suspended cells is compared to one of the cell lines (BHK, 3T3). The immunogenicity is evaluated *in vitro* and *in vivo*, as follows.

*In vitro* and *in vivo* studies were conducted in order to evaluate the biocompatibility of the encapsulated hMSCs in comparison to encapsulated HEK-293 cell line, a known and broadly used cell line in cell micro encapsulation.

***Encapsulated human MSCs are less-immunogenic than non-stem encapsulated cells*** - To test the immunogenecity of the encapsulated stem cells ex vivo, cells isolated from mouse spleens were cultured with microcapsules containing stem cells or control cells. As shown in Figures 14a-d, the encapsulated MSCs stimulate less the spleen cells as the levels of IL-1-β or TNF-α were lower than the levels in the encapsulated HEK 293 cells, demonstrating that they are less immunogenic than HEK 293 cells. The LPS was used to stimulate the spleen cells as a control group to the experiment.

***Encapsulated stem cells are less immunogenic in vivo than encapsulated non-stem cells*** - C57BL mice were inoculated with a right flank subcutaneous injection of microcapsules containing, hMSCs, rMSCs, or HEK-293 cell line or with empty microcapsules (devoid of cells). Mice were sacrificed at four time points following transplantation: one week, two weeks, four weeks and eight weeks. At each scarification time point the transplanted microcapsules and several organs were retrieved and taken for analysis.

***Encapsulated stem cells do not lead to a fibrotic reaction in vivo*** - Encapsulated stem cells were transplanted into mice and two months after transplantation the capsules were retrieved and the immunological reaction was evaluated. The microcapsules clusters morphology in their transplantation site was visualized post scarification. In the encapsulated rMSCs and hMSCs groups the transplanted microcapsules remained as a clear cluster with no severe tissue surrounding it (see for example, Figure 15b) while in the HEK-293 group intense vascularization was observed and the right inguinal lymph nodes was swallowed and irritated (Figure 15a). The retrieved microcapsules were viewed under light microscope for fibrosis or cellular overgrowth. The microcapsules entrapping the hMSCs and rMSCs were clean of tissue overgrowth (Figure 15d, and Figures 22a and c) while the microcapsules with the HEK-293 cells were entrapped within a capsular structure (Figure 15c, and Figure 22b).

***Encapsulated stem cells are viable after transplantation in vivo -*** Staining of the encapsulated cells with FDA for cell viability revealed that the cells were viable even after 8 weeks post transplantation (Figures 16a-c).

***Transplantation of encapsulated stem cells induces a reduced inflammatory reaction as compared to transplantation of encapsulated non-stem cells*** - The mice inguinal lymph nodes were homogenized, RNA samples were obtained and RT-PCR analysis was performed using primers specific for the IL-1β and TNF-α inflammatory factors (for primers' sequences see Table 1 above). The results show that in the first week post transplantation the levels of both IL-1β and TNF-α cytokines were high and during the second to the eight week post transplantation there was a decrease in the inflammation rate. In addition, the levels of the inflammatory cytokines were 2-3 folds lower in mice transplanted with encapsulated stem cells (hMSCs or rMSCs) as compared to mice transplanted with the encapsulated HEK-293 cells, similarly to the level observed in mice transplanted with empty capsules (Figures 17a and b).

Histochemistry and immunohistochemistry were performed on the retrieved microcapsules in order to analyze and characterize the immune reaction which was developed towards the microcapsules implant. Hematoxilin and Eosin (H&E) staining was used to visualize and evaluate the immune reaction towards the microcapsule implant. As can be seen in Figures 18a-c, in mice transplanted with the microcapsules containing HEK-293 cells a massive tissue was observed surrounding the microcapsules (Figure 18a). In comparison, in mice transplanted with the microcapsules containing hMSCs or rMSCs, a narrow layer of cells was observed (Figure 18c). In mice transplanted with the empty capsules the microcapsules were free of surrounding cells (Figure 18b).

### EXAMPLE 6

### DEVELOPMENT OF GENETICALLY MODIFIED HMSCS FOR THE TREATMENT OF GLIOMA CANCER

Genetically modified hMSCs were generated using lentivirus transduction. The lentivirus was packaged in 293FT cell line using the ViraPower^{™} Lentiviral Expression System and the expression vector: CSCW2-IRES-mCherry with or without the anti antigenic factor PEX. hMSCs were transduced with a viral supernatant from 293FT cells resulting in > 97 % efficiency (Figure 19). The expression of PEX in genetically modified hMSCs was confirmed by RT-PCR (Figure 20).

The bioactivity of PEX secreted from encapsulated hMSCs was evaluated on human glioma cell line (U-87). The proliferation assay showed that the encapsulated PEX expressing hMSCs inhibited the proliferation of U87 glioma cells by 47% (Figure 21).

### EXAMPLE 7

### DEPLETION OF CD34 CELLS FROM THE PREPARATIONS OF THE PRESENT INVENTION

Adherent cell population of hMSC were immunodepleted from hematopoietic cells (CD34+) using antibodies against CD34, and CD31 connected to microbeads from Miltenyi Biotec according to the manufacturer's instructions. Results of cell depletion are shown in Figure 23.

## Claims

1. A method of producing encapsulated primary mesenchymal stem cells comprising:
(a) providing a population of cells which comprise at least 97 % primary mesenchymal stem cells with an alginate to thereby obtain a solution of said alginate with said primary mesenchymal stem cells, and
(b) infusing droplets of said solution into a Calcium Chloride (CaCl₂) solution, to thereby generate spheres of said alginate and said mesenchymal stem cells,
(c) mixing said spheres with a poly-L lysine (PLL) solution, to thereby create semi permeable microcapsules,
thereby producing the encapsulated mesenchymal stem cells.

2. A composition-of-matter comprising an alginate poly-L lysine (PLL) microcapsule encapsulating primary mesenchymal stem cells generated according to the method of claim 1.

3. A method of ex-vivo proliferating and/or differentiating mesenchymal stem cells, comprising culturing the composition-of-matter of claim 2 under conditions required for the proliferation and/or differentiation of mesenchymal stem cells, thereby proliferating and/or differentiating mesenchymal stem cells.

4. The composition-of-matter of claim 2 for transplantation in a subject in need thereof.

5. A pharmaceutical composition comprising the composition-of-matter of claim 2 and a pharmaceutically acceptable carrier.

6. The composition-of-matter of claim 2 or 4, the method of claim 1 or 3, or the pharmaceutical composition of claim 5, wherein said mesenchymal stem cells do not express a heterologous polynucleotide.

7. The composition-of-matter of claim 2 or 4, the method of claim 1 or 3, or the pharmaceutical composition of claim 5, wherein said mesenchymal stem cells express a heterologous polynucleotide.

8. The composition-of-matter of claim 2 or 4, the method of claim 1 or 3, or the pharmaceutical composition of claim 5, wherein said mesenchymal stem cells being derived from a tissue selected from the group consisting of bone marrow, adipose tissue, embryonic yolk sac, placenta, umbilical cord and skin.

9. The composition-of-matter of claim 2 or 4, the method of claim 1 or 3, or the pharmaceutical composition of claim 5, wherein said mesenchymal stem cells express surface markers including CD105, CD90, CD44 and CD29 and not CD31, CD34, CD 144 and CD133.

10. The composition-of-matter of claim 2 or 4, the method of claim 1 or 3, or the pharmaceutical composition of claim 5, wherein said mesenchymal stem cells are capable of differentiating into osteoblasts, chondrocytes and adipocytes.

11. The composition-of-matter, the method, or the pharmaceutical composition of claim 7, wherein said heterologous polynucleotide comprises a therapeutic polynucleotide.

12. The composition-of-matter of claim 4, wherein said subject in need thereof has a medical condition selected from the group consisting of stem cell deficiency, heart disease, Parkinson's disease, cancer, Alzheimer's disease, stroke, burns, loss of tissue, loss of blood, anemia, autoimmune disorders, diabetes, arthritis, Multiple Sclerosis, graft versus host disease (GvHD), a neurodegenerative disorder, autoimmune encephalomyelitis (EAE), systemic lupus erythematosus (SLE), rheumatoid arthritis, systemic sclerosis, Sjorgen's syndrome, multiple sclerosis (MS), Myasthenia Gravis (MG), Guillain- Barré Syndrome (GBS), Hashimoto's Thyroiditis (HT), Graves's Disease, Insulin dependent Diabetes Melitus (IDDM) and Inflammatory Bowel Disease.

13. The composition-of-matter of claim 2 or 4, the method of claim 1 or 3, or the pharmaceutical composition of claim 5, wherein said primary mesenchymal stem cells maintain their proliferative capacity within said microcapsule.

14. The composition-of-matter, the method, or the pharmaceutical composition of claim 13, wherein said proliferative capacity is maintained after 90 days in culture.

## Patentansprüche

1. Verfahren zum Herstellen von verkapselten primären mesenchymalen Stammzellen, das umfasst:
(a) Versehen einer Population von Zellen, die zumindest 97 % primäre mesenchymale Stammzellen umfassen, mit einem Alginat, um eine Lösung des Alginats mit den primären mesenchymalen Stammzellen zu erhalten, und
(b) Infundieren von Tröpfchen der Lösung in eine Calciumchlorid-(CaCl₂-)Lösung, um Kügelchen des Alginats und der mesenchymalen Stammzellen zu erzeugen,
(c) Vermischen der Kügelchen mit einer Poly-L-Lysin-(PLL-)Lösung, um halbdurchlässige Mikrokapseln zu bilden,
wodurch die verkapselten mesenchymalen Stammzellen hergestellt werden.

2. Substanzzusammensetzung, die eine Alginat-Poly-L-Lysin-(PLL-)Mikrokapsel umfasst, in die primäre mesenchymale Stammzellen verkapselt sind, erzeugt gemäß dem Verfahren nach Anspruch 1.

3. Verfahren zum Ex-vivo-Proliferieren und/oder -Differenzieren von mesenchymalen Stammzellen, das das Züchten der Substanzzusammensetzung nach Anspruch 2 unter Bedingungen umfasst, die für die Proliferation und/oder Differenzierung von mesenchymalen Stammzellen erforderlich sind, wodurch mesenchymale Stammzellen proliferiert und/oder differenziert werden.

4. Substanzzusammensetzung nach Anspruch 2 zum Transplantieren in ein Individuum, das einer solchen bedarf.

5. Pharmazeutische Zusammensetzung, die die Substanzzusammensetzung nach Anspruch 2 und einen pharmazeutisch unbedenklichen Träger umfasst.

6. Substanzzusammensetzung nach Anspruch 2 oder 4, Verfahren nach Anspruch 1 oder 3 oder pharmazeutische Zusammensetzung nach Anspruch 5, wobei die mesenchymalen Stammzellen ein heterologes Polynukleotid nicht exprimieren.

7. Substanzzusammensetzung nach Anspruch 2 oder 4, Verfahren nach Anspruch 1 oder 3 oder pharmazeutische Zusammensetzung nach Anspruch 5, wobei die mesenchymalen Stammzellen ein heterologes Polynukleotid exprimieren.

8. Substanzzusammensetzung nach Anspruch 2 oder 4, Verfahren nach Anspruch 1 oder 3 oder pharmazeutische Zusammensetzung nach Anspruch 5, wobei die mesenchymalen Stammzellen von einem Gewebe abgeleitet sind, das aus der Gruppe ausgewählt ist, bestehend aus Knochenmark, Fettgewebe, embryonalem Dottersack, Plazenta, Nabelschnur und Haut.

9. Substanzzusammensetzung nach Anspruch 2 oder 4, Verfahren nach Anspruch 1 oder 3 oder pharmazeutische Zusammensetzung nach Anspruch 5, wobei die mesenchymalen Stammzellen Oberflächenmarker, darunter CD105, CD90, CD44 und CD29 und nicht CD31, CD34, CD144 und CD133, exprimieren.

10. Substanzzusammensetzung nach Anspruch 2 oder 4, Verfahren nach Anspruch 1 oder 3 oder pharmazeutische Zusammensetzung nach Anspruch 5, wobei die mesenchymalen Stammzellen in der Lage sind, sich zu Osteoplasten, Chondrozyten und Adipozyten zu differenzieren.

11. Substanzzusammensetzung, Verfahren oder pharmazeutische Zusammensetzung nach Anspruch 7, wobei das heterologe Polynukleotid ein therapeutisches Polynukleotid umfasst.

12. Substanzzusammensetzung nach Anspruch 4, wobei das Individuum, das einer solchen bedarf, einen medizinischen Zustand aufweist, der aus der Gruppe ausgewählt ist, bestehend aus Stammzelldefizienz, Herzkrankheit, Morbus Parkinson, Krebs, Morbus Alzheimer, Schlaganfall, Verbrennungen, Gewebeverlust, Blutverlust, Anämie, Autoimmunstörungen, Diabetes, Arthritis, multipler Sklerose, Transplantatversus-Wirt-Krankheit (GvHD), einer neurodegenerativen Störung, autoimmuner Enzephalomyelitis (EAE), systemischem Lupus erythematodes (SLE), rheumatoider Arthritis, systemischer Sklerose, Sjorgen-Syndrom, multipler Sklerose (MS), Myasthenia gravis (MG), Guillain-Barré-Syndrom (GBS), Hashimoto-Thyroiditis (HT), Morbus Grave, insulinabhängigem Diabetes mellitus (IDDM) und entzündlicher Darmkrankheit.

13. Substanzzusammensetzung nach Anspruch 2 oder 4, Verfahren nach Anspruch 1 oder 3 oder pharmazeutische Zusammensetzung nach Anspruch 5, wobei die primären mesenchymalen Stammzellen ihre Proliferationsfähigkeit innerhalb der Mikrokapsel aufrecht erhalten.

14. Substanzzusammensetzung, Verfahren oder pharmazeutische Zusammensetzung nach Anspruch 13, wobei die Proliferationsfähigkeit nach 90 Tagen in Kultur aufrechterhalten ist.

## Revendications

1. Procédé de production de cellules souches mésenchymateuses primaires encapsulées comprenant :
(a) la fourniture d'une population de cellules qui comprend au moins 97 % de cellules souches mésenchymateuses primaires avec un alginate de manière à obtenir une solution dudit alginate avec lesdites cellules souches mésenchymateuses primaires, et
(b) l'infusion de gouttelettes de ladite solution dans une solution de chlorure de calcium (CaCl₂), de manière à générer des sphères dudit alginate et desdites cellules souches mésenchymateuses,
(c) le mélanges desdites sphères avec une solution de poly-L-lysine (PLL), de manière à créer des microcapsules semi-perméables,
de manière à produire les cellules souches mésenchymateuses encapsulées.

2. Composition de matière comprenant une microcapsule d'alginate-poly-L-lysine (PLL) encapsulant des cellules souches mésenchymateuses primaires générée selon le procédé de la revendication 1.

3. Procédé de prolifération et/ou de différenciation *ex vivo* de cellules souches mésenchymateuses, comprenant la culture de la composition de matière de la revendication 2 dans des conditions requises pour la prolifération et/ou la différenciation de cellules souches mésenchymateuses, de manière à obtenir la prolifération et/ou la différenciation de cellules souches mésenchymateuses.

4. Composition de matière de la revendication 2 pour transplantation dans un sujet en ayant besoin.

5. Composition pharmaceutique comprenant la composition de matière de la revendication 2 et un véhicule pharmaceutiquement acceptable.

6. Composition de matière de la revendication 2 ou 4, procédé de la revendication 1 ou 3, ou composition pharmaceutique de la revendication 5, où lesdites cellules souches mésenchymateuses n'expriment pas un polynucléotide hétérologue.

7. Composition de matière de la revendication 2 ou 4, procédé de la revendication 1 ou 3, ou composition pharmaceutique de la revendication 5, où lesdites cellules souches mésenchymateuses expriment un polynucléotide hétérologue.

8. Composition de matière de la revendication 2 ou 4, procédé de la revendication 1 ou 3, ou composition pharmaceutique de la revendication 5, où lesdites cellules souches mésenchymateuses sont dérivées d'un tissu choisi dans le groupe constitué de la moelle osseuse, le tissu adipeux, le sac vitellin embryonnaire, le placenta, le cordon ombilical et la peau.

9. Composition de matière de la revendication 2 ou 4, procédé de la revendication 1 ou 3, ou composition pharmaceutique de la revendication 5, où lesdites cellules souches mésenchymateuses expriment des marqueurs de surface comprenant CD105, CD90, CD44 et CD29 et pas CD31, CD34, CD 144 et CD133.

10. Composition de matière de la revendication 2 ou 4, procédé de la revendication 1 ou 3, ou composition pharmaceutique de la revendication 5, où lesdites cellules souches mésenchymateuses sont capables de se différencier en ostéoblastes, chondrocytes et adipocytes.

11. Composition de matière, le procédé, ou la composition pharmaceutique de la revendication 7, où ledit polynucléotide hétérologue comprend un polynucléotide thérapeutique.

12. Composition de matière de la revendication 4, où ledit sujet en ayant besoin a une affection médicale choisie dans le groupe constitué d'une déficience en cellules souches, d'une maladie cardiaque, de la maladie de Parkinson, d'un cancer, de la maladie d'Alzheimer, d'un accident vasculaire cérébral, de brûlures, d'une perte de tissu, d'une perte de sens, d'une anémie, de troubles auto-immuns, du diabète, de l'arthrite, de la sclérose en plaques, d'une réaction de greffe contre hôte (GvHD), d'un trouble neurodégénératif, de l'encéphalomyélite auto-immune (EAE), du lupus érythémateux disséminé (SLE), de la polyarthrite rhumatoïde, de la sclérodermie généralisée, du syndrome de Sjorgen, de la sclérose en plaques (MS), de la myasthénie grave (MG), du syndrome de Guillain-Barre (GBS), de la thyroïdite de Hashimoto (HT), de la maladie de Graves, du diabète sucré insulinodépendant (IDDM) et d'un syndrome abdominal inflammatoire.

13. Composition de matière de la revendication 2 ou 4, procédé de la revendication 1 ou 3, ou composition pharmaceutique de la revendication 5, où lesdites cellules souches mésenchymateuses primaires maintiennent leur capacité proliférative dans ladite microcapsule.

14. Composition de matière, procédé, ou composition pharmaceutique de la revendication 13, où ladite capacité proliférative est maintenue après 90 jours en culture.
